# EUROPEAN PATENT SPECIFICATION

(11) **EP 3 575 793 B1**
(45) Date of publication and mention of the grant of the patent: **19.07.2023**
(21) Application number: 18174963.1
(22) Date of filing: 29.05.2018
(51) Int. Cl.: G01N 33/574, G01N 33/92

(54) **A METHOD OF DIAGNOSING CANCER BASED ON LIPIDOMIC ANALYSIS OF A BODY FLUID**
VERFAHREN ZUR KREBSDIAGNOSE DURCH LIPIDOMISCHE ANALYSE EINER KÖRPERFLÜSSIGKEIT
PROCÉDÉ DE DIAGNOSTIC DU CANCER BASÉ SUR L'ANALYSE LIPIDOMIQUE D'UN FLUIDE CORPOREL

(43) Date of publication of application: 04.12.2019
(73) Proprietor: FONS JK Group, a.s., 53002 Pardubice (CZ)
(72) Inventor: Holcapek, Michal, 53352 Stare Hradiste (CZ); Jirasko, Robert, 53002 Pardubice (CZ); Wolrab, Denise, 1030 Wien (AT); Cifkova, Eva, 75663 Krhova (CZ)
(74) Representative: Hartvichova, Katerina

(56) References cited:
- WO-A1-2015/104113
- WO-A1-2016/164474
- WO-A1-2017/155473
- WO-A1-2017/186859
- NIMA PATEL ET AL: "A Novel Three Serum Phospholipid Panel Differentiates Normal Individuals from Those with Prostate Cancer", PLOS ONE, vol. 9, no. 3, 6 March 2014 (2014-03-06), page e88841, XP055509802, DOI: 10.1371/journal.pone.0088841
- LOUBNA A. HAMMAD ET AL: "Elevated levels of hydroxylated phosphocholine lipids in the blood serum of breast cancer patients", RAPID COMMUNICATIONS IN MASS SPECTROMETRY., vol. 23, no. 6, 30 March 2009 (2009-03-30) , pages 863-876, XP055509806, GB ISSN: 0951-4198, DOI: 10.1002/rcm.3947
- LÍSA MIROSLAV ET AL: "Lipidomic analysis of biological samples: Comparison of liquid chromatography, supercritical fluid chromatography and direct infusion mass spectrometry methods", JOURNAL OF CHROMATOGRAPHY A, vol. 1525, 24 November 2017 (2017-11-24), pages 96-108, XP085274447, ISSN: 0021-9673, DOI: 10.1016/J.CHROMA.2017.10.022

## Description

### Field of Art

The present invention relates to a method of diagnosing kidney cancer based on lipidomic analysis of a body fluid, which is suitable for high throughput screening with a high sensitivity and specificity.

### Background Art

Cancer is one of the most serious human diseases, which results in huge number of deaths worldwide. There are numerous types of cancer, typically classified according to the primary organ, when the tumor cells start to grow. Even the cancer of one particular organ has several disease subtypes resulting in large complexity and heterogeneity of this disease, which requires a personalized treatment to increase the chances for a full or at least a partial recovery of individual patients. Early diagnosis of the cancer is the most critical issue investigated by many research teams throughout the world, because the early cancer diagnosis can significantly increase the chances for survival and recovery. Unfortunately, many cancer types have no or only minor symptoms at early stages, so the common situation is that the patient is diagnosed too late. The prognosis is much better when the disease is recognized earlier, but the currently available diagnostic methods are complex, expensive, and laborious, which is unsuitable for routine population screening. Therefore, there is a need to continuously develop new methods for screening and diagnosing cancer and for distinguishing among various types of cancer, which would be fast, economical, with a limited invasiveness, suitable for routine high-throughput screening, with a sufficient sensitivity and specificity, i.e., low percentage of false positive and false negative results.

Various methods based on specific biomarkers are investigated, using samples of body fluids, most commonly blood. These tentative biomarkers are often proteins, glycoproteins, products of protein cleavage, and short RNA, such as miRNA. The diagnosis using these biomarkers usually relies on immunochemical methods or PCR.

It would be desirable to develop diagnostic methods based on other methodologies and other types of biomarkers. These screening methods have to fulfil several requirements, such as available treatment for early diagnosed disease with significantly better prognosis compared to late diagnosis, sufficiently high sample throughput to be able to perform at least a partial population screening of individuals at higher risk, the screening price has to be acceptable for the healthcare system in relation to the benefits obtained by early diagnosis, etc. The blood or urine collection is a common part of the preventative healthcare. For the population screening, a high throughput methodology based on blood analysis is indispensable. Once the blood analysis gives a positive result, further investigations such as imaging methods, i.e. magnetic resonance spectroscopy, have to be done. However, even though such test is urgently needed, there are just limited studies so far for the analysis of cancer from blood mainly based on the analysis of DNA, RNA or proteins. Previous studies showed that lipids play a crucial role in cancer development, as demonstrated in the analysis of tumor tissues and cell lines [E. Cífková, M. Holčapek, M. Lisa, D. Vrána, J. Gatěk, B. Melichar, Anal. Bioanal. Chem. 407 (2015) 991-1002, E. Cífková, M. Lisa, R. Hrstka, D. Vrána, J. Gatěk, B. Melichar, M. Holčapek, Rapid Commun. Mass Spectrom. 31 (2017) 253-263], but not yet for collectable body fluids applicable for high-throughput screening. The first attempts towards the lipidomic analysis of human body fluids have been presented by Metanomics [US 2013/0140452, WO 2016/207391], but unfortunately sensitivities and specificities were too low for real-life applications.

Patel N. et al., PLOS ONE, 9(3): e88841, 2014; Hammad L.A. et al., Rapid Commun. Mass Spectrom. 2009; 23: 863-876; Lisa M., Journal or Chromatography A, 1525 (2017) 96-108 are representative documents disclosing the state of the art in the field of analysis of lipids in body samples by mass spectrometry.

### Summary of the Invention

The present invention provides a method using lipidomic analysis for the diagnosis of a kidney cancer. The major advantage of the present invention is that the lipidomic profiling works well for all cancer stages including the most early stages. The basic and rather simple hypothesis explaining the success of lipidomic analysis for cancer screening is that the tumor cells are so quickly divided that these cells require a large number of building blocks for the preparation of lipid bilayers of new tumor cells. This process is much faster than conventional cell division in the healthy organism, therefore the lipidomic composition of tumor and normal cell must be different as well, and these changes are also reflected in collectable body fluids.

The present invention aims at overcoming the problems and challenges of the current state of the art, and to provide a method of diagnosing kidney cancer from the analysis of biological fluids, said method being usable for routine high-throughput population screening.

The present invention thus provides a method of diagnosing kidney cancer based on lipidomic analysis of a body fluid sample taken from the body of a patient, comprising the steps of:
- spiking of the sample with a set of internal standards comprising at least one internal standard for each lipid class present in the sample,
- subsequently processing the sample by liquid-liquid lipidomic extraction or by solid phase lipidomic extraction,
- measurement of the processed sample by a mass spectrometry method,
- determining concentrations for at least 28 more preferably for all lipids present at a level above detection threshold of the mass spectrometry method, using the internal standards for the corresponding lipid classes for the quantitation, or using relative concentrations, or using ratios of concentrations and/or signal intensities,
- statistical evaluation of the determined concentrations of the lipids, said statistical evaluation determining the level of probability of the patient suffering from kidney cancer, based on the determined lipid concentrations compared to a cancerous pattern and a non-cancerous pattern, wherein the cancerous pattern and the non-cancerous pattern are determined by statistical analysis of the lipid concentrations for a group of known cancer samples and non-cancer samples; and wherein the statistical evaluation is done separately for males and females.

The method of the invention allows to distinguish between a healthy person and a person suffering from kidney cancer. The cancerous pattern indicates that the patient suffers from cancer.

The term "body fluid" includes body fluids selected from plasma, serum, blood, urine.

In preferred embodiments, the concentrations of at least 28, or at least 37, or at least 60, or at least 120, or at least 230, or at least 400, or at least 406, or at least 440, or at least 450, or at least 500 lipids are determined.

In one preferred embodiment, the concentrations of at least 28 lipids are determined when the body fluid is blood, plasma or serum. A particularly preferred set of at least 28 lipids is shown in Table 2 below. A more preferred set of lipids is shown in Table 1 below.

The patient is a mammal, preferably a human.

The internal standards must be lipid-type compounds, structurally close to the relevant lipid class. Typically, the internal standards are compounds having the structure (in particular the polar head structure) typical for the relevant lipid class but containing fatty acyls with shorter chains than naturally occurring lipids (e.g. chains 12:0 or 14:0) or fatty acyls with odd number of carbon atoms (e.g. chains 17:0, 17:1 or 19:1) or isotopically labelled analogues (e.g. D7-Chol, D7-CE 16:0).

The lipid classes are as follows:

| | |
|---|---|
| Cholesterylesters (CE) | Lysophosphatidylserines (LPS) |
| Ceramides (Cer) | Monoacylglycerols (MG) |
| Diacylglycerols (DG) | Phosphatidic acids (PA) |
| Dihexosylceramides (Hex2Cer) | Phosphatidylcholines (PC) |
| Hexosylceramides (HexCer) | Phosphatidylethanolamines (PE) |
| Cholesterol | Phosphatidylglycerols (PG) |
| Lysophosphatidic acids (LPA) | Phosphatidylserines (PS) |
| Lysophosphatidylcholines (LPC) | Sphingomyelins (SM) |
| Lysophosphatidylethanolamines (LPE) | Sulfatides, Sulfohexosylceramides (Sul or SulfoHexCer) |
| Lysophosphatidylglycerols (LPG) | Sulfodihexosylceramides (SulfoHex2Cer) |
| Gangliosides GM3 | Triacylglycerols (TG) |

An example of a set of internal standards (IS) is the following list:

| | | |
|---|---|---|
| D7-CE 16:0 | LPE 14:0 | PG 14:0/14:0 |
| Cer d18:1/12:0 | LPG 14:0 | PS 14:0/14:0 |
| DG 12:1/12:1 | LPA 14:0 | SM d18:1/12:0 |
| Hex2Cer d18:1/12:0 | MG 19:1 | SulfoHexCer d18:1/12:0 |
| HexCer d18:1/12:0 | PA 14:0/14:0 | TG 19:1/19:1/19:1 |
| D7-Chol | PC 14:0/14:0 | LPS 17:1 |
| LPC 17:0 | PE 14:0/14:0 | GM3 18:1/12:0 |

The notation of lipid compounds in this text is as follows: abbreviation of the class of the compounds, number of carbons: number of double bonds. The abbreviation of the class may be preceded by information about isotopic labeling, if relevant.

Due to the addition of the internal standard before sample processing, slight differences due to for instance pipetting errors can be compensated as the internal standard is affected in the same way as the target compounds. This ensures a better reliability of the quantitation of individual lipids.

Liquid-liquid lipidomic extractions are known in the art. Organic solvents may be used, selected from chlorinated alkanes, dialkyl ethers, alcohols, and water mixtures, which form bilayer systems containing organic and aqueous phases. Preferably, chloroform, methyl-tert-butyl ether, methanol, ethanol, propanol, and/or butanol are used. Most preferably, chloroform - methanol - water system is used, as described e.g. in E. Cífková, M. Holčapek, M. Lisa, D. Vrána, J. Gatěk, B. Melichar, Anal. Bioanal. Chem. 407 (2015) 991-1002; or in E. Cífková, M. Lisa, R. Hrstka, D. Vrána, J. Gatěk, B. Melichar, M. Holčapek, Rapid Commun. Mass Spectrom. 31 (2017) 253-263. The organic phase of liquid-liquid extraction system containing lipids is collected and used for further processing. Solid phase extractions (SPE) are known in the art. The sorbent of typically nonpolar material (reversed-phase) is used to capture lipids containing hydrophobic acyl chains. SPE column is first washed and conditions with weak eluting solvent (e.g., water), then the sample is loaded (e.g., urine), washed with water, and finally eluted with the strong solvent suitable for the elution of retained lipids, e.g., methanol, ethanol, propanol, or in the mixture with chlorinated solvents (dichloromethane, chloroform, etc.) or hydrocarbons (heptane, hexane, etc.). As the migration and solubility of the components of the sample are time-dependent, it is highly preferred that the extraction step is always performed in the same way for all samples measured in one batch, in particular that the extraction is performed over the same period of time for each sample. The organic phase is typically processed by evaporation of the organic solvent and re-dissolving the residue in a solvent or mixture of solvents with highest solubility for the target compounds before diluting in the solvent for mass spectrometry measurement. Suitable solvents for mass spectrometry measurements include chlorinated alkanes, alkanes and alcohols, preferably chloroform or dichloromethane, hexane and C1-C4 alcohols, most preferably a mixture of chloroform and 2-propanol (e.g., in volume ratio 1:1) or hexane : 2-propanol : chloroform 7:1.5:1.5 or chloroform - methanol - 2-propanol (1:2:4, v/v/v). Further components may be added, e.g., ammonium acetate, acetic acid, etc. Alkanes, unless defined otherwise, mean C1-C6 alkanes, preferably C1-C4 alkanes.

It is preferred to remove proteins from the sample during or after the extraction, as proteins may undesirably interfere with the analysis of lipids.

The mass spectrometry method is preferably selected from shotgun mass spectrometry, liquid chromatography - mass spectrometry (LC/MS), ultrahigh-performance liquid chromatography - mass spectrometry (UHPLC/MS), supercritical fluid chromatography - mass spectrometry (SFC/MS), ultrahigh-performance supercritical fluid chromatography - mass spectrometry (UHPSFC/MS), and matrix-assisted laser desorption/ionization mass spectrometry (MALDI/MS).

The method of the present invention benefits from the use of a pooled sample. A pooled sample is a sample prepared by mixing identical volumes of several samples, wherein the mixed samples include samples from cancer patients and healthy volunteers. The ratio of samples originating from males to samples originating from females should be approximately the same as in the measured batch of samples. The term "approximately the same" refers to deviation from the ratio of male:female in the measured batch at most by 20 %, preferably at most by 10 %, more preferably at most by 5 %. The pooled sample is preferably obtained by pooling 50-100 samples.

Concentrations of internal standards in the mixture were carefully optimized in order to be applicable for all mass spectrometry based methods used in this invention. The concentration of the internal standard should thereby be preferable in the concentration range of naturally occurring lipids. However, as the concentration range varies significantly within the lipid class, it is preferred to use the concentration between 10 and 100% of the concentration of the most abundant naturally occurring lipid species within the respective class, more preferably between 20 and 80%.

The pooled sample, processed in the same way as any other sample, i.e., spiked with internal standard mixture and subjected to liquid-liquid extraction and any other sample processing steps, is used for the full validation and quality control during the mass spectrometry measurements. The order of samples is randomized for the sample preparation and measurement to avoid measurement sections of non-cancerous and cancerous samples in a certain portion of the measurement sequence.

The pooled sample may be used for observing intra-day accuracy and intra-day precision, as well as inter-day accuracy and inter-day precision.

Furthermore, it is advantageous to prepare test samples or pooled samples spiked with varying concentrations of the internal standard mixture.

The limit of detection (LOD) of a lipid is preferably determined based on signal to noise ratio, e.g., such as S/N = 3.

The pooled sample spiked with varying concentrations of the internal standard mixture is used for determining the lower limit of quantitation (LLOQ) and the upper limit of quantitation (ULOQ), said limits corresponding to the first and the last points of linearity range of the calibration curve. The calibration curve is the dependence of signal on the concentration of a lipid. The linearity range of calibration curve is an interval from LLOQ to ULOQ, where the concentration of lipids can be determined.

The concentrations for all lipids present at a level above LLOQ and below ULOQ of the mass spectrometry method are also commonly called "lipidomic profile".

The quantitation (determining concentrations) for all lipids present at a level above LLOQ and below ULOQ of the mass spectrometry method, using the internal standards for the corresponding lipid classes for the quantitation, preferably comprises the following procedures and corrections:
- isotopic correction (deisotoping), i.e., correction of signal intensity of a lipid having due to isotopic contribution M+2 of another lipid with a molecular weight being 2 mass units lower than said lipid (e.g., isotopic interference from the lipid with one additional double bond) or due to isotopic contribution M+1 of another lipid with a molecular weight 1 mass unit lower than said lipid (e.g., interferences between PC and SM subgroups). The correction is done by subtracting calculated M+2 (or M+1) relative intensity based on known intensity in measured spectra.
- zero filling procedure in which the signals of lipid species which are not detected for particular sample, are replaced by 50 to 100 %, preferably by 60 to 100 %, more preferably by 60 to 70% or by 80 to 90 % or by 67 % or by 80 %, of the minimum concentration observed for said lipid species in all samples.

The accuracy and reliability of quantitation is ensured or improved by the following features:
- use of at least one exogenous lipid class internal standard for the quantitation of lipid species in each class,
- coelution and coionization of lipid (sub)class internal standards and analytes from the same lipid class using either lipid class separation chromatography method coupled to mass spectrometry (MS) or MS without any chromatographic separation. This co-processing is ensured by the addition of the internal standard before the sample is processed,
- use of the pooled sample spiked with the internal standards as quality control sample injected after a predetermined number of samples,
- measurement control by extracting the signal response of each internal standard in each sample and evaluation of signal response of those over the time period of the measurement sequence daily
- zero filling procedure,
- isotopic correction.

These features helps to control the quality of measurements by prevention of low reproducibility, lack of robustness, signal shifts over time, ion suppression/enhancement, carry-over effects, and response change of mass spectrometer due to contamination.

When several hundreds of lipids are determined in hundreds of samples, it may happen that the expected signal is not detected for some lipid molecules due to various reasons, and then the concentration should be reported as "below LLOQ" in accordance with established analytical practice. This well-established analytical approach does not work well in case of lipidomic quantitation, and the MDA based on such approach would provide poor resolution of healthy / disease groups, and may not provide acceptable sensitivity and specificity for the detection of cancer. This approach is adapted to cases in which all missing signals correspond to the situation that the true concentration of the analyte is below LLOQ, for example the determination of low number of analytes well separated by chromatography. However, the situation in lipidomic quantitation is very different, because ca. 150-400 lipids per sample are quantified and many of them (or all in case of shotgun) are co-eluting, which may result in the absence of signal due to other reasons than the concentration below LLOQ, mainly due to ion suppression or other reasons causing the signal drop, including the system contamination, mobile phase impurity, bleeding of chromatographic column, etc. The complexity of lipidomic analysis is enormous, and it is a natural situation that some signals are not properly recorded and processed regardless of the best analytical practice and careful analytical work. Therefore, we have developed a new zero filling approach where the values that are missing for any reason are replaced by 75 to 85 %, preferably by 80%, of the minimum concentration observed for this lipid species in all samples. This approach significantly improves the quality of subsequent statistical analysis. Our preliminary results show that when the zero-filling approach is neglected, the quality of cancer detection is significantly worse or impossible. Afterwards the average and the standard deviation of the duplicates are calculated.

The statistical evaluation is preferably performed by multivariate data analysis (MDA). The MDA methods may be non-supervised statistical methods such as principal component analysis (PCA) or supervised statistical methods such as orthogonal projections to latent structures discriminant analysis (OPLS-DA).

The "cancerous pattern" means the pattern of lipid concentrations typical for samples of a body fluid obtained from patients suffering from kidney cancer. It is usually obtained by statistical analysis of the samples obtained from patients suffering from kidney cancer versus samples obtained from healthy volunteers.

The statistical evaluation is done separately for males and females.

Preferably, the statistical evaluation involves
- data pre-processing such as centering, scaling, and/or transformation. The centering correlates the changes relative to the average of the data set. The scaling unifies the impact of different concentration ranges on the model (UV and Pareto scaling) so that low concentrations (maybe equally important) are not masked by very abundant concentrations. The logarithmic transformation assembles the data to the normal distribution. A proper pre-processing has a significant influence on the quality of results.
- PCA analysis used for identification of outliers, measurement failures as well as other influential factors. The PCA analysis may also visualize differences between groups as well as cluster the quality control (QC) samples in comparison to other samples.
- discrimination analysis (OPLS-DA) for group separation of cancer patients and healthy volunteers. The discrimination analysis is performed for males and females separately.
- assignment of the statistical parameters as well as the evaluation of the prediction power.

The statistical methods are first used to build statistical models (cancerous patterns, and non-cancerous patterns) based on lipidomic profiles of body fluids of healthy volunteers and cancer patients. These statistical models are then used for visualization of differences of the cancerous and non- cancerous pattern, as well as for the determination of the level of probability of the patient suffering from kidney cancer, or being healthy, based on the determined lipid concentrations resulting in a cancerous pattern or a non-cancerous pattern, respectively.

The sensitivity and specificity describe the prediction power of the method to correctly assign samples from healthy volunteers or cancer patients groups. The sensitivity and specificity values should be over 70%, preferably over 80% or over 85%, more preferably over 90% for samples of known and unknown classification. In some embodiments, the sensitivity and specificity values achieved are over 95% for samples of known and unknown classification.

The sample throughput of the methodology of the invention is at least 10,000 samples per year and one mass spectrometry apparatus at current level of automation of standard mass spectrometry laboratories.

Suitable sets of lipids to be determined for diagnosing the presence of the cancer are usually obtained by an analysis of all detectable lipids for a group of healthy volunteers and cancer patients. The lipids which are present in different amounts in the healthy vs. cancer samples are preferred for inclusion into the sets of lipids to be determined.

**Table 1. The following table shows a preferred set of lipids the concentrations of which are preferably determined in the present invention in blood samples. Optionally, the concentrations of further lipids may be determined.**

| Glycerophospholipids | | | | | |
|---|---|---|---|---|---|
| LPC 15:0 | PC 36:2 | PC O-36:5 | PE 37:3 | PE O-36:6 | PI 34:2 |
| LPC 16:0 | PC 36:3 | PC O-37:1 | PE 37:5 | PE O-36:7 | PI 34:3 |
| LPC 16:1 | PC 36:4 | PC O-37:2 | PE 37:6 | PE O-37:1 | PI 35:0 |
| LPC 18:0 | PC 36:5 | PC O-37:3 | PE 38:0 | PE O-37:2 | PI 35:1 |
| LPC 18:1 | PC 37:3 | PC O-38:2 | PE 38:1 | PE O-37:3 | PI 36:1 |
| LPC 18:2 | PC 37:4 | PC O-38:3 | PE 38:2 | PE O-38:0 | PI 36:2 |
| LPC 20:3 | PC 37:5 | PC O-38:4 | PE 38:4 | PE O-38:1 | PI 36:3 |
| LPC 20:4 | PC 37:6 | PC O-38:5 | PE 38:5 | PE O-38:2 | PI 36:4 |
| LPC 22:6 | PC 38:2 | PC O-38:6 | PE 38:6 | PE O-38:3 | PI 38:1 |
| LPC O-16:0 | PC 38:3 | PC O-39:2 | PE 38:7 | PE O-38:4 | PI 38:2 |
| LPE 16:0 | PC 38:4 | PC O-39:3 | PE 39:0 | PE O-38:5 | PI 38:3 |
| LPE 18:0 | PC 38:5 | PC O-40:5 | PE 39:4 | PE O-38:6 | PI 38:4 |
| LPE 18:1 | PC 38:6 | PC O-40:6 | PE 39:5 | PE O-39:0 | PI 38:5 |
| LPE 18:2 | PC 39:5 | PC O-40:9 | PE 39:6 | PE O-39:1 | PI 40:0 |
| LPE 20:4 | PC 39:6 | PE 32:1 | PE 40:4 | PE O-39:2 | PI 40:4 |
| LPE 22:5 | PC 40:4 | PE 33:1 | PE 40:5 | PE O-40:0 | PI 40:5 |
| LPE 22:6 | PC 40:5 | PE 33:2 | PE 40:6 | PE O-40:4 | PI 40:6 |
| PC 30:0 | PC 40:6 | PE 34:0 | PE 40:7 | PE O-40:5 | PS 34:0 |
| PC 32:0 | PC O-33:0 | PE 34:1 | PE 41:2 | PE O-40:6 | PS 36:1 |
| PC 32:1 | PC O-33:1 | PE 34:2 | PE 42:9 | PE O-40:7 | PS 36:4 |
| PC 32:2 | PC O-33:2 | PE 34:3 | PE O-33:1 | PE O-40:8 | PS 36:5 |
| PC 34:1 | PC O-34:1 | PE 35:1 | PE O-34:1 | PE O-40:9 | PS 37:0 |
| PC 34:2 | PC O-34:2 | PE 35:2 | PE O-34:2 | PE O-42:2 | PS 38:1 |
| PC 34:3 | PC O-34:3 | PE 35:3 | PE O-35:1 | PG 32:1 | PS 38:2 |
| PC 34:4 | PC O-35:1 | PE 35:5 | PE O-35:2 | PG 36:0 | PS 38:3 |
| PC 35:1 | PC O-35:2 | PE 36:1 | PE O-35:3 | PG 36:4 | PS 38:4 |
| PC 35:2 | PC O-35:3 | PE 36:2 | PE O-36:1 | PI 32:0 | PS 38:6 |
| PC 35:3 | PC O-36:1 | PE 36:3 | PE O-36:2 | PI 32:1 | PS 38:7 |
| PC 35:4 | PC O-36:2 | PE 36:4 | PE O-36:3 | PI 33:0 | PS 42:1 |
| PC 35:5 | PC O-36:3 | PE 36:5 | PE O-36:4 | PI 33:1 | |
| PC 36:1 | PC O-36:4 | PE 37:0 | PE O-36:5 | PI 34:1 | |

| Sphingolipids | | | | | |
|---|---|---|---|---|---|
| Cer 33:2 (OH) | Hex2Cer 33:2 (OH) | SM 34:0 | SM 41:3 | Sul 38:2 | Sul 42:3 (OH) |
| Cer 34:1 | Hex2Cer 34:1 | SM 34:1 | SM 42:1 | Sul 38:2 (OH) | SulfoHex2Cer 34:1 |
| Cer 34:2 | Hex2Cer 39:1 (OH) | SM 34:2 | SM 42:2 | Sul 40:0 (2OH) | SulfoHex2Cer 40:1 |
| Cer 34:3 | Hex2Cer 39:2 (OH) | SM 35:1 | SM 42:3 | Sul 40:0 (OH) | SulfoHex2Cer 42:1 |
| Cer 35:3 (OH) | Hex2Cer 40:1 | SM 36:0 | SM 42:4 | Sul 40:1 | SulfoHex2Cer 42:2 |
| Cer 36:2 | Hex2Cer 41:3 (OH) | SM 36:1 | SM 43:1 | Sul 40:1 (OH) | GM3 34:1 |
| Cer 36:3 | Hex2Cer 42:2 | SM 36:2 | SM 43:2 | Sul 40:2 | GM3 34:2 |
| Cer 36:4 | HexCer 33:2 (OH) | SM 37:0 | SM 43:3 | Sul 40:2 (OH) | GM3 36:1 |
| Cer 39:2 (OH) | HexCer 34:1 | SM 37:1 | Sul 32:1 (OH) | Sul 41:1 | GM3 36:2 |
| Cer 40:1 | HexCer 39:2 (OH) | SM 38:0 | Sul 34:0 (OH) | Sul 41:1 (OH) | GM3 38:1 |
| Cer 40:2 | HexCer 40:1 | SM 38:1 | Sul 34:1 | Sul 41:2 | GM3 38:2 |
| Cer 40:2 (OH) | HexCer 41:2 (OH) | SM 38:2 | Sul 34:1 (OH) | Sul 41:2 (OH) | GM3 40:2 |
| Cer 41:1 | HexCer 41:3 (OH) | SM 39:1 | Sul 34:2 | Sul 42:0 (2OH) | GM3 40:1 |
| Cer 41:2 (OH) | HexCer 42:1 | SM 39:2 | Sul 34:2 (OH) | Sul 42:1 | GM3 41:1 |
| Cer 41:3 (OH) | HexCer 42:2 | SM 40:0 | Sul 35:1 | Sul 42:1 (2OH) | GM3 41:2 |
| Cer 42: 1 | HexCer 42:2 (OH) | SM 40:1 | Sul 36:1 | Sul 42:1 (OH) | GM3 42:3 |
| Cer 42:2 | HexCer 43:1 | SM 40:2 | Sul 36:1 (OH) | Sul 42:2 | GM3 42:2 |
| Cer 42:2 (OH) | SM 32:1 | SM 40:3 | Sul 37:1 | Sul 42:2 (2OH) | GM3 42:1 |
| Cer 42:3 | SM 32:1 | SM 41:1 | Sul 38:1 | Sul 42:2 (OH) | GM3 42:2 (OH) |
| Cer 43:1 | SM 33:1 | SM 41:2 | Sul 38:1 (OH) | Sul 42:3 | GM3 42:1 (OH) |

| Glycerolipids | | | | | |
|---|---|---|---|---|---|
| MG 16:0 | DG 36:1 | TG 47:2 | TG 50:5 | TG 53:4 | TG 56:4 |
| MG 16:1 | DG 36:2 | TG 48:0 | TG 51:1 | TG 53:5 | TG 56:5 |
| MG 18:0 | DG 36:3 | TG 48:1 | TG 51:2 | TG 54:1 | TG 56:6 |
| MG 18:1 | DG 36:4 | TG 48:2 | TG 51:3 | TG 54:2 | TG 56:7 |
| MG 18:2 | TG 42:2 | TG 48:3 | TG 51:4 | TG 54:3 | TG 56:8 |
| MG 19:0 | TG 44:0 | TG 48:4 | TG 51:5 | TG 54:4 | TG 57:1 |
| DG 30:0 | TG 44:1 | TG 49:0 | TG 51:6 | TG 54:5 | TG 58:0 |
| DG 32:0 | TG 44:2 | TG 49:1 | TG 52:1 | TG 54:6 | TG 58:2 |
| DG 32:1 | TG 45:0 | TG 49:2 | TG 52:2 | TG 54:7 | TG 58:3 |
| DG 32:2 | TG 45:1 | TG 49:3 | TG 52:3 | TG 54:8 | TG 58:4 |
| DG 34:0 | TG 46:0 | TG 50:0 | TG 52:4 | TG 55:4 | TG 58:5 |
| DG 34:1 | TG 46:1 | TG 50:1 | TG 52:5 | TG 55:5 | TG 58:6 |
| DG 34:2 | TG 46:2 | TG 50:2 | TG 52:6 | TG 55:6 | TG 58:7 |
| DG 34:3 | TG 47:0 | TG 50:3 | TG 53:2 | TG 56:1 | TG 58:8 |
| DG 36:0 | TG 47:1 | TG 50:4 | TG 53:3 | TG 56:2 | TG 58:8 |

| Sterol lipids | | | | | |
|---|---|---|---|---|---|
| CE 14:0 | CE 16:1 | CE 18:2 | CE 20:4 | CE 22:6 | cholesterol sulfate |
| CE 16:0 | CE 18:1 | CE 18:3 | CE 20:5 | cholesterol | cholesterol sulfate (OH) |

**Table 2. The following table shows the most preferred set of lipids the concentrations of which are the most preferably determined in the present invention in the blood samples.**

| Sphingolipids | | | |
|---|---|---|---|
| Cer42: 1 | SM 38:1 | SM 42:1 | Sul 40:2 (OH) |
| SM 32:1 | SM 39:1 | SM 43:1 | Sul 41:1 |
| SM 33:1 | SM 40:1 | Sul 34:0 (OH) | Sul 41:1 (OH) |
| SM 34:2 | SM 40:2 | Sul 34:2 (OH) | Sul 42:1 |
| SM 36:2 | SM 41:1 | Sul 40:1 | Sul 42:1 (2OH) |
| SM 37:1 | SM 41:2 | Sul 40:1 (OH) | Sul 42:1 (OH) |

| Glycerophospholipids | | | |
|---|---|---|---|
| LPC 18:1 | LPC 18:2 | PC 32:2 | PC 36:3 |

**Table 3. The following table shows a preferred set of lipids the concentrations of which are preferably determined in the present invention for urine samples. Optionally, the concentrations of further lipids may be determined.**

| Sterol conjugates | Sulfoglycosphinglolipids | |
|---|---|---|
| hydroxypregnenolone sulfate | Sul 34:1 | Sul 42:1 (2OH) |
| lithocholic acid glycine conjugate | Sul 34:1 (OH) | Sul 42:0 (2OH) |
| cortisol sulfate | Sul 36:1 | Sul 43:1 (2OH) |
| glycochenodeoxycholic acid | Sul 36:1 (OH) | Sul 43:0 (2OH) |
| lithocholic acid sulfate | Sul 38:1 | SulfoHex₂Cer 34:1 |
| testosterone glucuronide | Sul 38:1 (OH) | SulfoHex₂Cer 34:1 (OH) |
| glycocholic acid | Sul 40:2 | SulfoHex₂Cer 36:1 |
| androsterone glucuronide / dihydrotestosterone glucuronide | Sul 40:1 | SulfoHex₂Cer 38:1 |
| | Sul 41:2 | SulfoHex₂Cer 38:1 (OH) |
| cholesterol sulfate | Sul 40:2 (OH) | SulfoHex₂Cer 40:2 |
| glycochenodeoxycholic acid sulfate | Sul 41:1 | SulfoHex₂Cer 40:1 |
| hydroxyandrostenedione glucuronide | Sul 40:1 (OH) | SulfoHex₂Cer 41: 1 |
| 11-oxo-androsterone glucuronide | Sul 40:0 (OH) | SulfoHex₂Cer 40:1 (OH) |
| hydroxyandrosterone glucuronide | Sul 42:3 | SulfoHex₂Cer 42:3 |
| taurolithocholic acid | Sul 42:2 | SulfoHex₂Cer 42:2 |
| sulfocholic acid | Sul 41:2 (OH) | SulfoHex₂Cer 42:1 |
| pregnanediol glucuronide | Sul 42:1 | SulfoHex₂Cer 41:1 (OH) |
| taurodeoxycholic acid | Sul 41:1 (OH) | SulfoHex₂Cer 40:0 (2OH) |
| sulfoglycolithocholic acid | Sul 41:0 (OH) | SulfoHex₂Cer 42:2 (OH) |
| taurocholic acid | Sul 40:0 (2OH) | SulfoHex₂Cer 42:1 (OH) |
| glycochenodeoxycholic acid sulfate | Sul 42:3 (OH) | SulfoHex₂Cer 41:0 (2OH) |
| aldosterone glucuronide | Sul 42:2 (OH) | SulfoHex₂Cer 42:0 (2OH) |
| tetrahydroaldosterone glucuronide | Sul 42:1 (OH) | |
| psychosine sulfate | Sul 42:0 (OH) | |
| deoxycholic acid glucuronide | Sul 41:0 (2OH) | |
| cholic acid glucuronide | Sul 43:2 (OH) | |
| glycochenodeoxycholic acid glucuronide | Sul 43:1 (OH) | |

Preferred sets of lipids for individual types of cancers are shown in the following tables.

**Table 4. Most up- and down-regulated species from S-plot of OPLS-DA model when comparing non-cancerous samples with cancerous samples for both genders and males (M) and kidney cancer (N=non-tumor, T=tumor, data about sensitivity and specificity relate to a set of persons whose samples were measured employing UHPSFC/MS).**

| S-plot | N-T kidney cancer M |
|---|---|
| Up-regulated species in cancer | DG 34:0 |
| | DG 36:0 |
| | MG 18:0 |
| | MG 16:0 |
| | TG 42:2 |
| | TG 49:0 |
| | Cer 42:3 |
| Down-regulated species in cancer | SM 42:1 |
| | SM 41:1 |
| | TG 58:6 |
| | LPC 18:2 |
| | SM 40:1 |
| | SM 38:1 |
| | SM 41:2 |
| | Cer 42: 1 |
| | SM 40:2 |
| | TG 58:4 |
| No.: N/T | 75/81 |
| Sensitivity [%] | 90.1 |
| Specificity [%] | 90.7 |

**Table 5. Most up- and down-regulated species from S-plot of OPLS-DA model when comparing non-cancerous samples with cancerous samples for females (F) (N=non-tumor, T=tumor, data about sensitivity and specificity relate to a set of persons whose samples were measured employing UHPSFC/MS).**

| S-plot | N-T kidney cancer F |
|---|---|
| Up-regulated species in cancer | MG 18:0 |
| | MG 16:0 |
| | DG 34:0 |
| | DG 36:0 |
| | - |
| | - |
| | - |
| Down-regulated species in cancer | SM 42:1 |
| | SM 41:1 |
| | SM 41:2 |
| | TG 56:1 |
| | PC 32:2 |
| | SM 40:1 |
| | Cer 42:1 |
| | TG 47:1 |
| | TG 49:1 |
| | TG 51:4 |
| No.: N/T | 95/31 |
| Sensitivity [%] | 74.2 |
| Specificity [%] | 100 |

**Table 6. Most up- and down-regulated species from S-plot of OPLS-DA model when comparing non-cancerous samples with cancerous samples for males (M) and kidney cancer (N=non-tumor, T=tumor, data about sensitivity and specificity relate to a set of persons whose samples were measured employing MALDI-MS).**

| S-plot | N-T kidney cancer M |
|---|---|
| Up-regulated | SulfoHex2Cer 42:2 |
| Down-regulated species in cancer | SM 43:1 |
| | Sul 41:1 (OH) |
| | SM 39:1 |
| | Sul 40:1 (OH) |
| | Sul 34:0 (OH) |
| | Sul 40:1 |
| | SM 41:1 |
| | Sul 42:1 (OH) |
| | SM 32:1 |
| | Sul 34:2 (OH) |
| No.: N/T | 75/81 |
| Sensitivity [%] | 86.4 |
| Specificity [%] | 93.3 |

**Table 7. Most up- and down-regulated species from S-plot of OPLS-DA model when comparing non-cancerous samples with cancerous samples for females (F) (N=non-tumor, T=tumor, data about sensitivity and specificity relate to a set of persons whose samples were measured employing MALDI-MS).**

| S-plot | N-T kidney cancer F |
|---|---|
| Up-regulated | SulfoHex2Cer 42:2 |
| Down-regulated species in cancer | Sul 41:1 (OH) |
| | Sul 34:0 (OH) |
| | SM 39:1 |
| | SM 43:1 |
| | Sul 40:1 (OH) |
| | Sul 42:1 (OH) |
| | Sul 42:1 (2OH) |
| | Sul 41:1 |
| | SM 41:1 |
| | Sul 40:1 |
| No.: N/T | 95/31 |
| Sensitivity [%] | 77.4 |
| Specificity [%] | 99.0 |

**Table 8. Most up- and down-regulated species from S-plot of OPLS-DA model when comparing non-cancerous samples with cancerous samples for males (M) (N=non-tumor, T=tumor, data about sensitivity and specificity relate to a set of persons where results from MALDI and UHPSFC are combined).**

| S-plot | N-T kidney cancer M |
|---|---|
| Up-regulated species in cancer | DG 34:0 |
| | DG 36:0 |
| | MG 18:0 |
| | MG 16:0 |
| | SulfoHex2Cer |
| | 42:2 |
| | TG 49:0 |
| | TG 42:2 |
| | PI 38:6 |
| Down-regulated species in cancer | SM 42:1 |
| | TG 58:6 |
| | SM 41:1 |
| | Sul 41:1 (OH) |
| | SM 40:1 |
| | SM 43:1 |
| | SM 38:1 |
| | Sul 40:1 (OH) |
| | SM 39:1 |
| | SM 41:2 |
| No.: N/T | 75/81 |
| Sensitivity [%] | 93.8 |
| Specificity [%] | 93.3 |

**Table 9. Most up- and down-regulated species from S-plot of OPLS-DA model when comparing non-cancerous samples with cancerous samples for females (F) (N=non-tumor, T=tumor, data about sensitivity and specificity relate to a set of persons where results from MALDI and UHPSFC are combined).**

| S-plot | N-T kidney cancer F |
|---|---|
| Up-regulated species in cancer | MG 18:0 |
| | MG 16:0 |
| | DG 34:0 |
| | DG 36:0 |
| | Cer 42:3 |
| | SulfoHex₂Cer 42:2 |
| | - |
| | - |
| Down-regulated species in cancer | SM 42:1 |
| | SM 41:1 |
| | Sul 41:1 (OH) |
| | SM 41:2 |
| | SM 39:1 |
| | Sul 42:1 (OH) |
| | Sul 40:1 (OH) |
| | TG 51:4 |
| | TG 49:2 |
| | PC 32:2 |
| No.: N/T | 95/31 |
| Sensitivity [%] | 83.9 |
| Specificity [%] | 100 |

**Table 10. Most up- and down-regulated species from S-plot of OPLS-DA model when comparing non-cancerous plasma samples with kidney cancerous plasma samples for males (M) and females (F) (N=non-tumor, T=tumor, data about sensitivity and specificity relate to a set of persons whose samples were measured employing shotgun MS).**

| S-plot | N-T kidney F | N-T kidney M |
|---|---|---|
| Up-regulated species in cancer | PE 42:9 | LPC 16:0 |
| | SM 42:2 | SM 42:2 |
| | PC 38:4 | Hex2Cer 34:1 |
| | MG 19:0 | PS 42:1 |
| | LPC O-16:0 | PS 36:5 |
| | TG 56:5 | HexCer 42:2 |
| | PS 32:2 | CE 16:0 |
| | SM 36:0 | PC 34:1 |
| | CE 16:0 | PS 36:4 |
| | LPC 18:0 | MG 18:0 |
| | CE 14:0 | LPC 18:2 |
| | PC 32:2 | PE P-36:4 |
| | SM 33:1 | SM 39:1 |
| | SM 32:1 | PC 34:4 |
| | PC 34:4 | SM 41:2 |
| | SM 39:1 | PC O-36:5 |
| | SM 41:1 | PS 38:7 |
| Down-regulated species in cancer | PC 34:3 | SM 41:1 |
| | HexCer 42:1 | TG 56:6 |
| | LPC 18:2 | SM 40:2 |
| No.:T/N | 28/31 | 71/30 |
| Sensitivity [%] | 100 | 95.8 |
| Specificity [%] | 93.6 | 76.7 |

**Table 11. Most up- and down-regulated species from S-plot of OPLS-DA model when comparing non-cancerous (N) urine samples with kidney cancerous (T) urine samples for both genders, males (M) and females (F) (N=non-tumor, T=tumor, data about sensitivity and specificity relate to a set of persons whose samples were measured employing MALDI-MS.**

| S-plot | N-T kidney cancer both genders | N-T kidney cancer M | N-T kidney cancer F |
|---|---|---|---|
| Up-regulated species in cancer | SulfoHex2Cer 42:2 | SulfoHex2Cer 40:2 | Sul 42:3 |
| | Sul 42:3 | Sul 42:3 | SulfoHex2Cer 40:2 |
| | SulfoHex2Cer 40:2 | SulfoHex2Cer 38:1 | SulfoHex2Cer 42:2 |
| | SulfoHex2Cer 38:1 | sulfocholic acid | SulfoHex2Cer 38:1 (OH) |
| | SulfoHex2Cer 42:1 | SulfoHex2Cer 42:2 | SulfoHex2Cer 42:1 |
| | cortisol sulfate | SulfoHex2Cer 38:1 (OH) | Sul 43:1 (2OH) |
| | SulfoHex2Cer 40:1 | SulfoHex2Cer 34:1 | Sul 42:3 (OH) |
| | SulfoHex2Cer 34:1 | SulfoHex2Cer 42:1 | SulfoHex2Cer 40:1 |
| | SulfoHex2Cer 38:1 (OH) | SulfoHex2Cer 34:1 (OH) | Sul 42:2 |
| | sulfocholic acid | Sul 43:1 (2OH) | SulfoHex2Cer 41:1 (OH) |
| Down-regulated species in cancer | taurolithocholic acid | Sul 40:0 (2OH) | taurolithocholic acid |
| | Sul 40:0 (2OH) | Sul 41:0 (2OH) | taurocholic acid |
| | Sul 41:0 (2OH) | Sul 41:0 (OH) | taurodeoxycholic acid |
| | Sul 38:1 (OH) | Sul 38:1 (OH) | sulfoglycolithocholic acid |
| | cholesterol sulfate | Sul 40:0 (OH) | Sul 40:0 (2OH) |
| | Sul 41:0 (OH) | Sul 40:1 (OH) | Sul 34:1 (OH) |
| | Sul 40:0 (OH) | Sul 42:0 (2OH) | Sul 41:0 (2OH) |
| | SulfoHex2Cer 40:0 (2OH) | Sul 42:1 (2OH) | Sul 38:1 (OH) |
| | Sul 42:0 (2OH) | Sul 41:1 (OH) | Sul 40:0 (OH) |
| | Sul 40:1 (OH) | Sul 41:1 | Sul 42:1 (2OH) |
| No.: N/T | 70/101 | 34/72 | 36/29 |
| Sensitivity [%] | 87.1 | 91.7 | 86.2 |
| Specificity [%] | 87.1 | 76.5 | 86.1 |

### Brief description of Drawings

Figure 1: OPLS-DA plot for the analysis of non-cancerous and cancerous samples suffering from kidney cancer for males.
Figure 2: OPLS-DA plot for the analysis of non-cancerous and cancerous samples suffering from kidney cancer for females.

T1, T2, T3, T4 are stages of cancer (primary tumor) according to TNM classification. T1 is a very early stage of cancer.

### Detailed description of the Invention

### Sample collection:

Human body fluid samples of cancer patients and healthy volunteers are collected in the hospital, typically blood or urine. Other samples types are isolated from blood, such as plasma, serum. The blood of human subjects is collected in the standard and well established way used in hospitals into anticoagulant-containing tubes, such as EDTA, heparin, citrate tubes, then serum or plasma is isolated using standardized protocols well known to a person skilled in the art, storage at -80°C at the clinic, transport from the clinic to the analytical laboratory using biological transport bags with dry ice (-20°C), storage again at -80°C until the analysis.

### Preparation of a set of internal standards (mixture of internal standards):

Generally, an internal standard mixture is used for quantitation of lipid species. The internal standard for each lipid class behaves in the same way as the target compounds belonging to this lipid class. Due to the addition of the internal standard before sample processing, slight differences due to for instance pipetting errors can be compensated as the internal standard is affected in the same way as the target compounds. In a particular embodiment, 2-4 mg of each standard is weighed into 2 mL HPLC glass vials using an analytical balance and dissolved in corresponding volume (chloroform : 2-propanol - 2:8) in order to obtain a final concentration of 0.25, 1, 2, or 2.1µg/µL. Standards for each lipid class are mixed together to form internal standard mixtures as described in the following table for all herein shown types of MS analysis.

Preferably, one mixture of internal standards is prepared in a sufficient amount for the whole experiment, in order to avoid variances in quantitation due to slight differences in concentration of the internal standards, when the mixture is prepared multiple times for individual batches. Aliquots may be prepared of this internal standard mixture and stored at -80°C.

**Table 12. Preparation of internal standards mixture.**

| **Internal standards** | **MW** | **stock concentration [µg/µL]** | **Volume [µL]** | **Concentration in body fluid [nmol/mL]** |
|---|---|---|---|---|
| CE d7 16:0 | 631.6285 | 2 | 200 | 443.3 |
| Cer d18:1/12:0 | 481.4495 | 2 | 4 | 11.6 |
| DG 12:1/12:1 | 452.3502 | 2 | 15 | 46.4 |
| Hex2Cer d18:1/12:0 | 805.5551 | 2 | 4 | 7.0 |
| HexCer d18:1/12:0 | 643.5023 | 2.1 | 3 | 6.9 |
| Chol d7 | 393.6988 | 2 | 300 | 1066.8 |
| PI 33:1 d7 | 846.596 | 1 | 16 | 13.2 |
| LPC 17:0 | 509.3481 | 2.1 | 20 | 57.7 |
| LPE 14:0 | 425.2542 | 2 | 4 | 13.2 |
| LPG 14:0 | 478.2308 | 2.1 | 14 | 43.0 |
| MG 19:1 | 370.3083 | 2 | 30 | 113.4 |
| PA 14:0/14:0 | 614.3924 | 2 | 4 | 9.1 |
| PC 14:0/14:0 | 677.4996 | 2 | 60 | 124.0 |
| PE 14:0/14:0 | 635.4526 | 2 | 4 | 8.8 |
| PG 14:0/14:0 | 688.4291 | 2 | 2 | 4.1 |
| PS 14:0/14:0 | 701.4244 | 2 | 4 | 8.0 |
| SM d18:1/12:0 | 646.505 | 2 | 20 | 43.3 |
| SulfoHexCer d18:1/12:0 | 740.4857 | 0.25 | 0.28 | 0.1 |
| TG 19:1/19:1/19:1 | 926.8302 | 2 | 75 | 113.3 |
| PC 44:2 | 897.719 | 2 | 80 | 124.8 |
| Solvent | | CHCl₃:IPA 2:8 | 140.72 | |
| Total Volume | | | 1000 | |

### Sample processing:

All samples are spiked before homogenization and extraction with the appropriate internal standard mixture.

The lipidomic extractions were preferably performed by well-established procedures using chloroform - methanol - water extraction systems as published in our previous works [E. Cífková, M. Holčapek, M. Lisa, D. Vrána, J. Gatěk, B. Melichar, Anal. Bioanal. Chem. 407 (2015) 991-1002; E. Cífková, M. Lisa, R. Hrstka, D. Vrána, J. Gatěk, B. Melichar, M. Holčapek, Rapid Commun. Mass Spectrom. 31 (2017) 253-263]. The order of samples for extractions is randomized.

25 µL of serum or plasma and 17.5 µL of internal standard mixture and 2 mL chloroform and 1 mL methanol are transferred into a glass vial (4 mL). Biological samples contain also proteins, which may lead to sticky and slimy samples. It must be ensured that the pipette draws the 25 µL of serum or plasma and is not blocked with the slimy components during drawing. The glass vials are closed and put for 10 min into the ultrasonic bath at 40°C for homogenization. Afterwards, the samples are allowed to reach room temperature in order to ensure no evaporation of the organic solvents when opening the vials. 600 µL of water are added to each sample. The samples are closed and vortexed for 1 min. It is important to do the extraction step always in the same way for all samples of one batch (one study). The target lipids for mass spectrometry are better soluble in the organic layer (bottom layer). Afterwards, the samples are centrifuged for 3 min at 3000 rpm in order to separate the organic and aqueous layer (in between these two layers a protein layer in the form of white precipitate is formed). The aqueous layer (upper layer) is removed via a glass pipette. The organic solvent containing the target compounds is evaporated under a stream of nitrogen at 30 °C and the glass vials containing the residue (target compounds) are stored at - 80°C. Before analysis, the samples are allowed to reach ambient temperature (so that they cannot draw water from the air when opening the vials) and then the residue from the extraction and evaporation (previous step) is dissolved in 500 µL chloroform : 2-propanol (1:1, v/v). It is advisable to prepare enough solvent mixture of chloroform : 2-propanol (1:1, v/v) mixture for all samples to avoid variations between individual batches of the solvent mixture. The samples are vortexed carefully for 1 min to ensure that the target compounds are dissolved. The solution is filtered using a 0.2 µm syringe filter in order to get rid of undissolved components, which may compromise the MS analysis. The vials are closed with PTFE caps and stored at -80°C.

For urine samples, reversed-phase solid phase extraction is performed. 2 mL of human urine together with 3 µL of mixture of internal standards dissolved in methanol (SufoHexCer d18:1/12:0 of concentration 1.7 µg/mL and D4 taurocholic acid of concentration 16.7 µg/mL) are loaded on 200 mg tC18 cartridge (Sep-Pak Vac, 37-55 µm particle size) (Waters, Milford, MA, USA) previously primed with 3 mL of methanol followed by 3 mL of water. Columns are washed with 3 mL of water, and studied lipids are further eluted with 3 mL of methanol. The eluates are collected, then evaporated by the gentle stream of nitrogen and redissolved in the mixture of 300 µL of methanol before the measurement.

### Sample preparation for UHPSFC/MS analysis

The filtrate is diluted 5 times or 20 times with a mixture of hexane : 2-propanol : chloroform 7:1.5:1.5 and transferred into an HPLC vial. The vials containing the diluted filtrate are closed with slit caps for analysis with UHPSFC/MS and placed in the autosampler.

### Sample preparation for shotgun MS analysis

The filtrates are diluted 10 times depending on the samples by chloroform - methanol - 2-propanol (1:2:4, v/v/v) mixture containing 7.5 mmol/L of ammonium acetate and 1% of acetic acid.

### Sample preparation for MALDI-MS analysis

MALDI matrix 9-aminoacridine (Sigma-Aldrich, St. Louis, MO, USA) is dissolved in methanol - water mixture (4:1, v/v) to provide the concentration of 5 mg/ml or 10 mg/ml and mixed with particular lipid extracts (1:1, v/v) - less preferably lipid extracts can be diluted with methanol (1:1, 1:2, or 1:3, v/v) before mixing with matrix. The deposited amount of extract/matrix mixture is 1 µl and the dried droplet crystallization is used for the sample deposition on the target plate. The deposition of small aliquot of chloroform on MALDI plate spots before the application of diluted extract/matrix mixture is applied to avoid the drop spreading.

### Mass spectrometry (MS) method development and validation

Three major MS based methods for lipidomic quantitation were developed in particular, which are described in more detail here. The method benefits from the use of pooled sample, which is a mixture of identical volumes of all samples for smaller studies with less than 100 subjects. In this study, the pooled sample is prepared from equal volumes of randomly selected cancer patients suffering from different types of cancer and healthy volunteers samples, keeping the ratio of males and females in the same proportion as in the sample set. The pooled sample is used for the method development and optimization. The pooled sample with and without added internal standard mixture per each lipid class to be quantified is used for the full validation and QC during the measurements. The order of samples is always randomized in sample sequences to avoid measurements of non-cancerous and cancerous samples in certain portion of sequence.

The system suitability test was carried out before the validation procedure at three concentration levels typically reported as low, medium and high concentration levels. All concentration levels must be within the linear dynamic range. The low concentration level is close to the lower limit of quantitation (LLOQ), the middle concentration level is in the middle of the linear dynamic range, and the high concentration level is close to the upper limit of quantitation (ULOQ). In a particular embodiment, we use 5, 17.5, and 30 µl of IS mixture prepared as described above for low, medium and high concentration levels, respectively. Validation parameters such as selectivity, accuracy, precision, calibration curve, limits of detection and quantitation, matrix effect, carry-over and stability were determined in line with recommendations of authoritative organizations, such Food and Drug Administration (FDA) or European Medicines Agency (EMA). Individual parameters were determined for IS representing properties of the lipid class. The selectivity was determined by comparing the results obtained for 3 extracts of the pooled serum samples spiked before extraction with the IS mixture at low, middle and high concentration level and 3 extracts of appropriate non-spiked serum samples, in order to show that the internal standard mixture can be measured in the body fluid as they are not naturally occurring. The accuracy, repeatability and precision were studied using the pooled serum sample spiked before or after the extraction at low, medium and high concentration levels. The accuracy and intermediate precision were studied using three samples per concentration level and two independent operators for sample preparation. The repeatability was determined by multiple injections of the same sample. The inter-day accuracy and inter-day precision were evaluated among three independent runs on two different days using three samples at the low, medium and high concentration level. The LLOQ and ULOQ corresponded to the first and the last points of linearity range, respectively. The limit of detection (LOD) was determined based on signal to noise ratio (*S*/*N* = 3) observed from reconstructed ion chromatogram or neutral loss (NL) and precursor ion (PI) mass spectra (shotgun MS) of internal standard mixture. The extraction recovery was determined by calculating the ratio of the signal response of samples spiked before and after extraction for low, medium and high concentration. The process efficiency was determined by calculating the ratio of the signal response of the spiked samples before extraction and the neat standard at different concentrations. The matrix effect was calculated from the ratio of the signal response of samples spiked after extraction and the neat standard. The carry-over was evaluated for each IS by the injection of blank sample with the pure solvent after the calibration sample at high concentration level (dilution factor of 10). The reliability of results obtained within analysis of large sample sets was evaluated by on-instrument and freeze-and-thaw stability tests. The stability of spiked plasma extract at middle concentration level was measured in autosampler at certain time intervals: 0, 4, 8, 12, 16, and 24 hours. Sample for freeze-and-thaw experiment was analyzed immediately after complete unassisted thawing in autosampler.

**Table 13. Dilution scheme for calibration with pooled sample for UHPSFC/MS volumes in µl.**

| Mixture of IS | Pooled sample | Hexane : 2-propanol : chloroform (7:1.5:1.5, v/v/v) |
|---|---|---|
| 150 | 100 | 250 |
| 100 | 100 | 300 |
| 50 | 100 | 350 |
| 20 | 100 | 380 |
| 15 | 100 | 385 |
| 10 | 100 | 390 |
| 5 | 100 | 395 |
| 3.5 | 100 | 396.5 |
| 2 | 100 | 398 |
| IS mixture 1 : 50 diluted | | |
| 75 | 100 | 325 |
| 50 | 100 | 350 |
| 25 | 100 | 375 |
| 10 | 100 | 390 |
| 7.5 | 100 | 392.5 |
| 5 | 100 | 395 |
| 2.5 | 100 | 397.5 |
| 1 | 100 | 399 |

For calibration, the optimized mixture of IS for all methods in several dilutions were used in order to calibrate in the concentration ranges relevant for individual lipid classes. Depending on the dilution factor of the corresponding mass spectrometric method, the corresponding amounts of matrix were used. For instance, UHPSFC/MS uses 1: 5 dilution of sample extracts. Therefore, 115 of blank plasma of a pooled sample were added.

### UHPSFC/MS

Supercritical fluid chromatography is a tool for the separation of compounds of different polarity employing supercritical carbon dioxide (mobile phase) as the main component for removing the compounds from an adsorbent (column - stationary phase). The addition of an organic solvent (typically methanol) to the supercritical carbon dioxide broadens the application range of UHPSFC and allows the removal of more polar compounds from the column. Generally, compounds can be differentiated, if they are better soluble in water or alcohols, than they are of polar nature or if they are better soluble for instance in hexane than they are of nonpolar nature. Depending on the nature of the stationary phase, the mobile phase and the target compounds, like polar or nonpolar, interactions can be forced. For instance, polar compounds prefer to interact with polar stationary phases and in order to remove those from the stationary phase, a polar mobile phase can be used to remove the compounds from the stationary phase. A careful adjustment of these interactions by optimization of the mobile phase properties using a certain stationary phase allows the separation of compounds. The optimization of dimensions and properties of the stationary phase, such as smaller particle size and spherical particles of the sorbents, allows for a higher efficiency and is therefore called ultrahigh performance supercritical fluid chromatography (UHPSFC).

The chromatographic separation can be optimized for the separation of lipid classes, taking into account in particular the following. A lipid class has a dominant structural moiety (polar head group) in common, which is mainly responsible for the interaction governing the retention mechanism. A lipid class can comprise numerous lipid species varying in the hydrocarbon chain length and structure (e.g., double bonds). Internal standards are added for each lipid (sub)class, therefore it is possible to identify and quantify all lipid species within a particular lipid (sub)class by comparing it to the class internal standard. MS analysis: Using a high-resolution, accurate-mass spectrometer as a detector allows the identification and quantification of lipids, as each lipid species has a defined m/z value and gives a signal response depending on the concentration in the sample. In order to improve ionization and therefore the signal response of the target compounds, additives like acids and buffers are added to the sample or to the mobile phase. In case of UHPSFC/MS, the use of a make-up solvent like acidified methanol further improves the sensitivity.

In one particular embodiment, a detailed description of all parameters applied for the UHPSFC/MS method to obtain the results presented herein below is as follows: Instrument - Acquity Ultra Performance Convergence Chromatography (UPC²) System hyphenated to the hybrid quadrupole-traveling wave ion mobility- time of flight mass spectrometer Synapt G2 Si from Waters. Chromatographic settings - stationary phase: Acquity BEH UPC² column (100 × 3 mm, 1.7 µm, Waters), the flow rate was 1.9 mL/min, the injection volume 1 µL, the column temperature 60°C, the active back pressure regulator (ABPR) was set to 1800 psi, gradient mode : CO₂ and methanol with 30 mM ammonium acetate and 1 % water. The gradient started at 1% modifier and increased to 51 % in 5 min, afterwards kept constant for 1 min and flushed back to starting conditions with a total run time of 7.5 min. injection needle wash: a mixture of hexane - 2-propanol - water (1:2:0.5, v/v) column wash after each biological sample injection: a blank was injected using a fast gradient: 0 min - 1%, 1.4 min - 51%, 1.6 min - 51%, 1.8 min - 1%, and 4.8 min - 1% modifier, make-up effluent: HPLC 515 pump (Waters), make-up flow rate 0.25 mL/min methanol with 1 % water and 0.1% formic acid.

ESI - MS settings: a capillary voltage of 3 kV, a sampling cone of 20 V, the source offset of 90 V, a source temperature of 150 °C, a drying temperature of 500°C, the cone gas flow of 50 L h⁻¹, the drying gas flow of 1000 L h⁻¹, and the nebulizer gas of 4 bar. Resolution mode or sensitivity mode in positive ion mode and a mass range of m/z 50-1200. The scan time was 0.15 s, and measurements were performed in the continuum mode. The peptide leucine enkephaline was used as the lock mass with a scan time of 0.1 s and interval of 30 s.

During the analysis, the samples in the sequence should be randomized so that not the same type of samples, such as only healthy (non-cancerous) samples, are measured in a row. This guarantees that in case of an error at a certain time not only one type of sample is affected. Furthermore, it is important to measure QC samples after a predetermined amount of samples in order to verify the instrument performance.

Before measuring biological samples, no injection, blank and QC samples are measured to check the instrument performance and afterwards it is continued with biological samples. All samples are measured in duplicates and after 20 samples or 40 + 40 injections (sample + wash) respectively, QC and blank samples are measured. During the whole study, measurement and sample preparation control is performed by evaluating the peak areas of each internal standard of each sample and exporting results in the Microsoft Excel file. The QC samples are aliquots of an extract of a mixture of serum or plasma samples. The lock mass is continuously measured during the analysis, however the lock mass correction is not applied online, as preliminary results showed that the mass accuracy is worse using the online correction. Furthermore, the continuum mode is applied so that it is possible to monitor the resolution of the instrument. After measurements, the raw data get noise reduced using the MassLynx software from Waters. This improves the mass spectra as well as significantly reduces the file size, which allows easier handling of the files for further processing. The files are further processed by applying the lock mass correction and converting the files from profile to centroid mode, which enhances the mass accuracy and further reduces the file size. The file size is important for data processing. The processing software can only hardly deal with huge file sizes and sample numbers, resulting in continuous errors making the processing time-consuming and cumbersome.

All investigations regarding measurement control, profile mode and offline lock mass correction as well as the noise reduction improved data quality.

### Shotgun Nrs

Experiments as presented herein below were performed on a quadrupole-linear ion trap mass spectrometer 6500 QTRAP (Sciex, Concord, ON, Canada) equipped by ESI probe with the following setting of tuning parameters: the ionspray voltage 5200 V, the curtain gas 20 psi, the source temperature 50°C, the ion source gas(*1*) 15 psi, and the ion source gas(2) 10 psi. MS/MS scans are measured with the scan rate 1000 Dais, the declustering potential 80 V, the entrance potential 10 V, and the collision energy specified in the Table 17. Samples are introduced by a flow injection using a liquid chromatograph Agilent 1290 Series (Agilent Technologies) consisted of Agilent 1290 binary pump and Agilent 1260 autosampler. 50 µL of sample was injected into the flow rate 3 µL/min of chloroform - methanol - 2-propanol (1:2:4, v/v/v) mixture containing 7.5 mmol/L of ammonium acetate and 1% of acetic acid with the analysis time 12 min, and the autosampler temperature 20°C. LC/MS system is washed after each analysis with methanol - 2-propanol - water (2:2: 1, v/v/v) mixture containing 7.5 mmol/L of ammonium acetate and 1% of acetic acid. Measured data experiments are extracted using LipidView software with the mass tolerance 0.3 Da, the minimum *S*/*N* = 5, and the minimum intensity 1%. Raw data characterized by type of scans, m/z values, and peak intensities are exported as *.txt* data and further processed using our Microsoft Excel macro script for the detection and quantitation of lipids. Lipid classes are characterized using the type of scans, and individual lipid species in selected MS/MS scan are detected according to *m*/*z* values with the mass tolerance 0.3 Da based on the database compiled from identified lipids in the pooled sample followed by the isotopic correction of ion intensities. The concentration of lipid species are calculated from corrected ion intensity related to the intensity of lipid class internal standards.

**Table 14. Characterization of shotgun MS/MS scans and their parameters for individual lipid classes. PI - precursor ion scan and NL - neutral loss scan.**

| **Lipid class** | **Type of MS/MS scan** | **Collision energy** | **Observed ions** |
|---|---|---|---|
| CE | PI 369; PI 376 | 12 | [M+NH₄]⁺ |
| Cer | PI 264; PI 266 | 35 | [M+H]⁺ |
| DG | NL 35 | 20 | [M+NH₄]⁺ |
| Hex2Cer | PI 264; PI 266 | 35 | [M+H]⁺ |
| HexCer | PI 264; PI 266 | 35 | [M+H]⁺ |
| Cholesterol | PI 369; PI 376 | 12 | [M+NH₄]⁺ |
| LPA | NL 115 | 25 | [M+NH₄]⁺ |
| LPC | PI 184 | 35 | [M+H]⁺ |
| LPE | NL 141 | 30 | [M+H]⁺ |
| LPG | NL 189 | 30 | [M+NH₄]⁺ |
| LPS | NL 185 | 30 | [M+H]⁺ |
| MG | NL35 | 20 | [M+NH₄]⁺ |
| PA | NL 115 | 25 | [M+NH₄]⁺ |
| PC | PI 184 | 35 | [M+H]⁺ |
| PE | NL 141 | 30 | [M+H]⁺ |
| PG | NL 189 | 30 | [M+NH₄]⁺ |
| PS | NL 185 | 30 | [M+H]⁺ |
| SM | PI 184 | 35 | [M+H]⁺ |
| SulfoHexCer | NL 98 | 25 | [M+H]⁺ |
| TG | NL 17 | 30 | [M+NH₄]⁺ |

### MALDI mass spectrometry

Mass spectra were measured using ultrahigh-resolution MALDI mass spectrometer LTQ Orbitrap XL (Thermo Fisher Scientific, Waltham, MA, USA) equipped with the nitrogen UV laser (337 nm, 60 Hz) with a beam diameter of about 80 µm × 100 µm. The LTQ Orbitrap instrument is operated in the negative-ion mode over a normal mass range m/z 400 - 2000 and the mass resolution is set to R = 100,000 (full width at half maximum definition, at *m*/*z* 400). The zig-zag (or spiral outwards) sample movement with 250 µm step size is used during the individual data acquisition. The laser energy corresponds to 15% of maximum and 2 microscans/scan with 2 laser shots per microscan at 36 different positions are accumulated for each measurement to achieve a reproducible signal. Each sample (spotted matrix and body fluid extract mixture) is spotted five times. The total acquisition time of one sample including five consecutive spots is around ten minutes. Each measurement is represented by one average MALDI-MS spectrum with thousands of *m*/*z* values. The automatic peak assignment is subsequently performed and particular *m*/*z* peaks are matched with deprotonated molecules from a database created during the identification procedure using the Excel macro script. This peak assignment results in the generation of the list of present *m*/*z* of studied lipids with the average intensities in particular spectra for each samples that is used for further statistical evaluation.

The values of intensities below LLOQ (minimum value of statistical confidence level) are subsequently replaced by 2/3 of lowest quantified value in order to unify statistically insignificant or zero values and enable further processing based on logarithmic calculations. Corrected intensities are subsequently normalized to IS or particular ratios of intensities within one lipid class are generated. Finally, the statistical evaluation and comparison of normalized data set is performed.

### Data processing and quantitation

The data processing starts with the data export from MS vendor software (e.g., Waters, Sciex, or Thermo Scientific) into a data-processing software, which may be Microsoft Excel for further steps to be done semi-automatically using e.g. advanced Excel script, in particular isotopic correction and zero-filling. Quality control (QC) samples should be regularly injected to check the right and constant response of mass spectrometer. The typical QC sample is a pooled sample containing internal standards for all lipid classes to be quantified, which is injected after every 20 injections, and responses of individual internal standards are plotted versus the time. If responses of the internal standards are reduced too much, then it is the indication of an instrumental problem, typically the mass spectrometer requires cleaning due to the injection of too many samples. The typical cleaning interval is about several hundreds of samples, but it may strongly depend on the quality of prepared sample extracts, geometry of ion source, system configuration, quality of solvents and additives used in the mobile phase.

The following example describes the example of UHPSFC/MS measurement on Synapt G2Si instrument from Waters, but similar approach is also applicable for other MS methods and different MS platforms from any instrumental vendor. The noise reduced, lock mass corrected and converted files are further processed with the MarkerLynx software. The reduced sequence table only including plasma and serum samples and QC samples has to be prepared in MassLynx with the corresponding suffix in the sample name (sample_nr2O_AFAMM). Then the time scan range for each lipid class over the whole sequence has to be determined (for example m/z 250-350 is used for CE). For each lipid class, the method is created in MarkerLynx with the corresponding scan range, which will be combined, the mass peak separation of 50 mDa and marker intensity threshold of 3000. The method for each lipid class is applied for the sequence and MarkerLynx table with m/z values against the combined intensities is created. This table is exported into a text file and imported into a homemade database for the identification and quantitation of lipid species using Microsoft Excel. The m/z values obtained from MarkerLynx are compared to the accurate m/z values deposited as database for hundreds of lipid species for the identification. The database was created by evaluating present species in tissue and body fluid samples. The identified lipid species are isotopically corrected and quantified by calculating the concentration in relation to the intensity and concentration of the (sub)class IS obtaining the table of lipid species concentrations vs. individual samples. Zero-filling procedure is performed. The average of the multiple injected samples for individual lipid species is calculated. The final data matrix, where the columns are individual subjects and lines are individual lipids, are used for further MDA statistical evaluation for absolute quantification. For relative quantification, the concentration of individual species within a class is related to the sum of concentrations of this lipid class in one sample. The resulting table is than used for statistical evaluation equally as for the absolute quantification.

Database of lipid species monitored during the identification step in the positive-ion mode together with the calculation of isotopic correction for M+1 and M+2 isotopic peaks (so called deisotoping) is described in EP 18152687 (Table 5) and can be used in the present method.

Database of lipid species monitored during the identification step in the negative-ion mode together with the calculation of isotopic correction for M+1 and M+2 isotopic peaks (so called deisotoping) is described in EP 18152687 (Table 6) and can be used in the present method.

### Statistical evaluation

Measured concentrations of individual lipids of all measured subjects are imported into a statistical software (e.g., SIMCA from Umetrics, Sweden). Proper transformation and scaling are chosen, typically logarithmic transformation and Pareto or UV scaling. The scaling and transformation are based on PCA analysis, where the normal distribution of healthy and cancer patients is desirable. PCA analysis is also used to find potential outliers, if so, the influence of the outlier on the model is tested (remove the outlier and check the model), and measurement methods are questioned. If a technical problem in case of outlier measurement is identified, then this measurement is removed from the data set. PCA method is used for finding other influential factors, such as gender or age. QC samples should cluster closely together in PCA analysis, typically close to the middle of the PCA graph.

The next step is the use of discrimination analysis (OPLS-DA) for the group separation of cancer patients and healthy volunteers separately for males and females. The scaling and transformation is based on PCA results, but the final model has to be found. Models are fitted on all data together and also on separate strata for influential covariates. Different groups of lipids are used to build the model and to explore the influence on the healthy vs. cancer separation. The final model is chosen based on multiple factors as good fit (all known samples are correctly classified), on the model stability (not too influential observations), good prediction ability (by cross-validation performed automatically and then manually with random groups of observations) and biological reasoning and resistance to removing unimportant lipids. The final models are used to identify unknown samples, and the sensitivity and specificity are estimated based on these predictions. For this purpose, the receiver operating characteristic (ROC) curves are plotted, and the area under curve (AUC) is calculated. The model is again tested for good prediction ability via the final classification. After this last validation of the model, the most dysregulated lipids are identified using the S-plot or the loading plot. For these most dysregulated lipids, the box-plots comparing the average values in the group of cancer and healthy patients for different strata are used to find exact biological interpretation.

### Results of experiments:

The present invention allows to determine whether the tested patient suffers from cancer, i.e., to distinguish between a healthy person and a person suffering from cancer.

### Example A: UHPSFC/MS lipidomic analysis of human plasma of cancer patients and healthy volunteers

Body fluid samples of various patients suffering from kidney cancer and healthy volunteers were analyzed for their lipid profile using UHPSFC/MS. Generally, absolute and relative concentrations of the lipid species can be used to determine differences in samples of different health state. In the following, absolute concentrations were used for statistical analysis in order to visualize differences between sample groups and errors in sample preparation or measurements. After centering, transformation and Pareto scaling, PCA of all samples were performed in order to identify outliers and measurement errors.

If unsupervised PCA does not show any significant clustering according to undesirable parameters (e.g., day of analysis) and unexplained outliers, then supervised OPLS-DA analysis is performed to improve the statistical model applicable also for predictions. If unwanted clustering or outliers are identified, then further investigations are necessary to identify the reason of those.

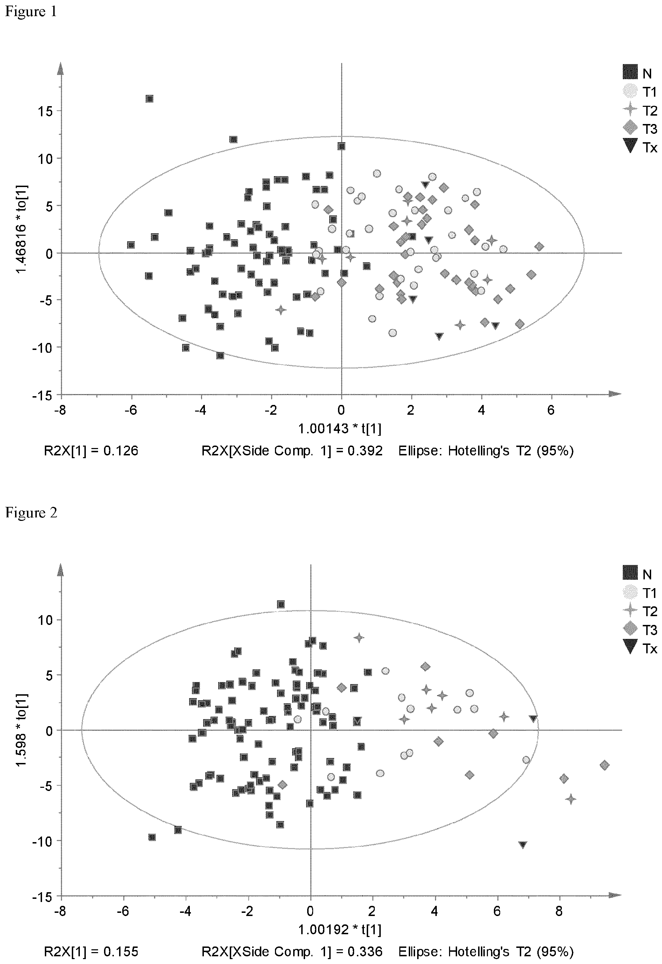

For OPLS-DA analysis, samples have to be defined according to their classification, i.e., groups of non-cancerous and cancerous samples. OPLS-DA analysis allows the visualization of differences between sample groups. It is preferred to have a clear differentiation, which means a gap between both groups. However, it may happen that there is a small overlay of both groups, as differences in the lipid pattern in biological fluids are not large enough due to the biological variability of samples or the investigated cancer type does not show a sufficiently large difference in the lipid profile measured in the biological fluid. Figure 1 shows the OPLS-DA plot for the analysis of non-cancerous and cancerous samples suffering from kidney cancer for males. The sensitivity for the analysis of kidney cancer samples from males is 90.1% and the specificity is 90.7%.

Figure 2 represents the OPLS-DA plot for the analysis of non-cancerous and cancerous samples suffering from kidney cancer for females. The sensitivity is 74.2 % and the specificity is 100% for the analysis of kidney cancer samples from females.

**Table 15. Statistical parameters for UHPSFC/MS analysis of non- cancerous plasma samples and cancerous plasma samples of males (M) and females (F) for kidney cancer.**

| | Fold change | | P-value | | T-value | |
|---|---|---|---|---|---|---|
| Species | F | M | F | M | F | M |
| TG 48:2 | 0.4 | 0.5 | 1.0E-07 | 7.7E-04 | 5.5 | 3.3 |
| TG 50:3 | 0.6 | 0.6 | 2.2E-07 | 4.9E-06 | 5.5 | 4.6 |
| TG 50:2 | 0.6 | 0.6 | 1.0E-07 | 1.2E-04 | 5.6 | 3.8 |
| TG 51:4 | 0.5 | 0.6 | 1.3E-05 | 1.6E-05 | 4.5 | 4.3 |
| TG 51: 3 | 0.6 | 0.6 | 9.4E-08 | 8.6E-07 | 5.8 | 5.1 |
| TG 51:2 | 0.5 | 0.5 | 1.2E-08 | 1.0E-05 | 6.1 | 4.5 |
| TG 52:3 | 0.7 | 0.7 | 1.3E-04 | 2.4E-06 | 3.9 | 4.8 |
| TG 53:4 | 0.6 | 0.6 | 4.5E-05 | 2.3E-05 | 4.2 | 4.3 |
| TG 53:3 | 0.6 | 0.6 | 9.8E-06 | 2.9E-05 | 4.6 | 4.2 |
| Cer40:1 | 0.6 | 0.7 | 2.8E-04 | 3.4E-03 | 3.7 | 2.8 |
| Cer42:1 | 0.5 | 0.6 | 1.0E-06 | 1.8E-04 | 5.3 | 3.7 |
| PC 32:2 | 0.4 | 0.7 | 8.3E-07 | 1.1E-02 | 5.1 | 2.3 |
| PC 34:2 | 0.6 | 0.7 | 7.6E-09 | 6.6E-07 | 6.7 | 5.1 |
| PC 34:1 | 0.6 | 0.7 | 1.9E-06 | 4.0E-03 | 5.2 | 2.7 |
| PC O-36:4 | 0.7 | 0.8 | 2.4E-03 | 2.0E-02 | 3.0 | 2.1 |
| PC O-36:3 | 0.6 | 0.8 | 8.1E-06 | 6.4E-02 | 4.6 | 1.5 |
| PC 36:4 | 0.7 | 0.7 | 1.6E-04 | 5.9E-04 | 3.9 | 3.3 |
| PC 36:3 | 0.6 | 0.7 | 8.9E-08 | 3.3E-06 | 6.0 | 4.7 |
| PC 36:2 | 0.6 | 0.6 | 6.7E-08 | 1.6E-07 | 6.1 | 5.4 |
| PC 38:6 | 0.7 | 0.8 | 2.3E-02 | 7.4E-02 | 2.1 | 1.5 |
| PC 38:5 | 0.7 | 0.7 | 1.2E-02 | 8.1E-03 | 2.4 | 2.4 |
| PC 38:4 | 0.8 | 0.7 | 1.1E-02 | 3.0E-05 | 2.4 | 4.2 |
| PC 38:3 | 0.7 | 0.7 | 4.7E-03 | 9.2E-05 | 2.7 | 3.9 |
| SM 34:2 | 0.7 | 0.7 | 3.3E-03 | 1.3E-04 | 2.9 | 3.8 |
| SM 34:1 | 0.6 | 0.6 | 2.6E-06 | 4.8E-09 | 5.1 | 6.1 |
| SM 36:2 | 0.8 | 0.7 | 3.0E-02 | 1.9E-03 | 2.0 | 3.0 |
| SM 36:1 | 0.8 | 0.7 | 6.9E-02 | 1.9E-03 | 1.5 | 3.0 |
| SM 38:2 | 0.7 | 0.8 | 1.1E-02 | 1.8E-02 | 2.4 | 2.1 |
| SM 38:1 | 0.6 | 0.6 | 4.7E-05 | 7.2E-06 | 4.3 | 4.5 |
| SM 40:2 | 0.6 | 0.6 | 2.3E-04 | 1.2E-05 | 3.8 | 4.4 |
| SM 40:1 | 0.6 | 0.6 | 1.0E-04 | 4.7E-07 | 4.1 | 5.2 |
| SM 41:2 | 0.5 | 0.6 | 4.9E-06 | 4.6E-06 | 5.0 | 4.6 |
| SM 41:1 | 0.5 | 0.5 | 3.5E-07 | 3.0E-08 | 5.6 | 5.7 |
| SM 42:3 | 0.8 | 0.7 | 4.2E-02 | 4.3E-03 | 1.8 | 2.7 |
| SM 42:2 | 0.7 | 0.6 | 3.7E-03 | 1.7E-05 | 2.9 | 4.3 |
| SM 42:1 | 0.4 | 0.5 | 4.7E-05 | 2.3E-03 | 4.2 | 2.9 |
| LPC 16:0 | 0.7 | 0.7 | 8.9E-03 | 4.9E-04 | 2.5 | 3.4 |
| LPC 18:2 | 0.4 | 0.5 | 1.2E-08 | 8.7E-07 | 6.1 | 5.0 |
| LPC 18:1 | 0.6 | 0.8 | 7.1E-04 | 2.3E-02 | 3.4 | 2.0 |
| LPC 18:0 | 0.7 | 0.7 | 2.5E-02 | 3.4E-04 | 2.0 | 3.5 |

**Table 16. Statistical parameters for MALDI-MS analysis of non- cancerous plasma samples and cancerous plasma samples of males (M) and females (F) for kidney cancer.**

| Species | Fold change | | P-value | | T-value | |
|---|---|---|---|---|---|---|
| | F | M | F | M | F | M |
| cholesterol sulfate | 0.57 | 0.80 | 5.1E-09 | 1.6E-02 | 6.3 | 2.2 |
| SM 32:1 | 0.54 | 0.60 | 4.3E-10 | 1.4E-11 | 7.4 | 7.2 |
| SM 33:1 | 0.57 | 0.63 | 1.4E-08 | 4.4E-10 | 6.6 | 6.6 |
| SM 34:2 | 0.77 | 0.71 | 3.7E-03 | 6.7E-06 | 2.9 | 4.5 |
| SM 34:1 | 0.72 | 0.75 | 3.4E-04 | 3.0E-05 | 3.7 | 4.2 |
| SM 34:0 | 0.68 | 0.70 | 7.8E-05 | 1.2E-06 | 4.2 | 5.0 |
| SM 35:1 | 0.64 | 0.66 | 1.1E-05 | 4.1E-08 | 4.8 | 5.7 |
| SM 36:2 | 0.86 | 0.74 | 7.2E-02 | 1.8E-04 | 1.5 | 3.7 |
| SM 36:1 | 0.81 | 0.74 | 1.5E-02 | 2.5E-05 | 2.3 | 4.2 |
| SM 37:1 | 0.66 | 0.65 | 7.0E-05 | 1.1E-07 | 4.2 | 5.5 |
| SM 38:2 | 0.76 | 0.69 | 2.7E-03 | 4.0E-06 | 3.0 | 4.7 |
| SM 38:1 | 0.65 | 0.64 | 1.8E-05 | 9.9E-10 | 4.7 | 6.5 |
| SM 39:1 | 0.52 | 0.53 | 3.4E-08 | 2.1E-14 | 6.4 | 8.4 |
| SM 40:2 | 0.65 | 0.64 | 1.0E-05 | 2.2E-08 | 4.8 | 5.8 |
| SM 40:1 | 0.65 | 0.63 | 2.5E-05 | 6.1E-10 | 4.6 | 6.5 |
| SM 41:2 | 0.60 | 0.64 | 5.2E-07 | 1.3E-08 | 5.6 | 5.9 |
| SM 41:1 | 0.58 | 0.59 | 1.3E-06 | 2.8E-12 | 5.5 | 7.5 |
| SM 42:1 | 0.61 | 0.62 | 4.6E-06 | 9.0E-10 | 5.1 | 6.5 |
| SM 43:3 | 0.65 | 0.75 | 2.1E-06 | 3.4E-05 | 5.0 | 4.1 |
| SM 43:2 | 0.62 | 0.64 | 3.1E-05 | 2.9E-06 | 4.4 | 4.7 |
| SM 43:1 | 0.39 | 0.39 | 8.2E-10 | 1.8E-11 | 6.6 | 7.4 |
| Sul 32:1 (OH) | 0.63 | 0.81 | 6.0E-07 | 6.9E-04 | 5.2 | 3.3 |
| Sul 34:1 | 0.62 | 0.68 | 5.6E-05 | 2.0E-05 | 4.3 | 4.3 |
| Sul 34:1 (OH) | 0.61 | 0.68 | 1.6E-05 | 2.9E-05 | 4.7 | 4.2 |
| Sul 34:0 (OH) | 0.43 | 0.58 | 4.9E-08 | 3.5E-05 | 6.1 | 4.1 |
| Sul 40:1 | 0.62 | 0.60 | 4.6E-04 | 1.1E-05 | 3.6 | 4.4 |
| Sul 41:1 | 0.49 | 0.60 | 4.2E-07 | 2.7E-05 | 5.5 | 4.2 |
| Sul 40:1 (OH) | 0.57 | 0.58 | 9.1E-06 | 5.1E-08 | 4.8 | 5.6 |
| Sul 41:1 (OH) | 0.50 | 0.52 | 3.6E-07 | 6.4E-09 | 5.7 | 6.1 |
| Sul 40:0 (2OH) | 0.71 | 0.88 | 1.7E-05 | 3.4E-02 | 4.3 | 1.8 |
| Sul 42:1 (OH) | 0.58 | 0.61 | 8.5E-06 | 1.2E-07 | 4.9 | 5.5 |
| Sul 42:1 (2OH) | 0.55 | 0.60 | 8.8E-04 | 1.1E-04 | 3.3 | 3.8 |
| Sul 42:0 (2OH) | 0.72 | 0.85 | 8.2E-05 | 2.2E-02 | 3.9 | 2.0 |
| SulfoHex₂Cer 42:2 | 1.34 | 1.84 | 9.1E-02 | 2.7E-02 | -1.4 | -2.0 |
| PI 36:4 | 0.59 | 0.82 | 2.7E-05 | 4.9E-02 | 4.3 | 1.7 |
| PI 36:3 | 0.54 | 0.75 | 8.6E-06 | 1.3E-02 | 4.6 | 2.3 |
| PI 36:2 | 0.62 | 0.81 | 6.5E-06 | 1.2E-02 | 4.7 | 2.3 |
| PI 36:1 | 0.53 | 0.70 | 4.7E-07 | 5.0E-04 | 5.4 | 3.4 |
| PI 38:5 | 0.52 | 0.74 | 7.1E-07 | 1.1E-02 | 5.3 | 2.3 |
| PI 38:3 | 0.60 | 0.72 | 6.6E-06 | 7.3E-04 | 4.8 | 3.3 |

**Table 17. Statistical parameters for the MALDI-MS analysis of non-cancerous urine samples and cancerous urine samples of males (M) and females (F) for kidney cancer.**

| Species | Fold change | | Kidney P-value | | T-value | |
|---|---|---|---|---|---|---|
| | F | M | F | M | F | M |
| cortisol sulfate | 1.9 | 3.8 | 5.2E-02 | 1.3E-03 | -1.7 | -3.1 |
| lithocholic acid sulfate | 1.4 | 2.4 | 2.0E-01 | 4.4E-02 | -0.9 | -1.7 |
| cholesterol sulfate | 1.1 | 1.7 | 3.8E-01 | 1.3E-02 | -0.3 | -2.3 |
| taurolithocholic acid | 0.6 | 1.6 | 1.0E-01 | 9.7E-02 | 1.3 | -1.3 |
| sulfocholic acid | 2.3 | 6.6 | 5.7E-02 | 5.7E-03 | -1.7 | -2.6 |
| taurodeoxycholic acid | 0.8 | 2.8 | 3.3E-01 | 8.6E-03 | 0.5 | -2.4 |
| sulfoglycolithocholic acid | 1.0 | 1.6 | 4.4E-01 | 2.9E-02 | 0.2 | -1.9 |
| taurocholic acid | 0.6 | 2.0 | 8.6E-02 | 1.3E-02 | 1.4 | -2.3 |
| glycochenodeoxycholi c acid sulfate | 1.0 | 2.3 | 4.8E-01 | 1.5E-02 | 0.0 | -2.2 |
| psychosine sulfate | 1.1 | 1.4 | 3.8E-01 | 5.2E-02 | -0.3 | -1.7 |
| Sul 34:1 (OH) | 1.1 | 1.0 | 3.8E-01 | 4.4E-01 | -0.3 | -0.1 |
| Sul 36:1 (OH) | 1.7 | 1.8 | 7.8E-02 | 3.9E-03 | -1.5 | -2.8 |
| Sul 38:1 | 1.2 | 1.0 | 3.2E-01 | 4.9E-01 | -0.5 | 0.0 |
| Sul 38:1 (OH) | 1.1 | 0.7 | 4.3E-01 | 1.3E-01 | -0.2 | 1.1 |
| Sul 40:2 | 1.6 | 1.5 | 1.8E-01 | 8.2E-02 | -1.0 | -1.4 |
| Sul 41:2 | 1.8 | 1.9 | 7.9E-02 | 1.8E-03 | -1.5 | -3.0 |
| Sul 41:1 | 0.9 | 1.0 | 4.1E-01 | 4.5E-01 | 0.2 | 0.1 |
| Sul 40:1 (OH) | 0.8 | 0.9 | 3.0E-01 | 3.4E-01 | 0.5 | 0.4 |
| Sul 40:0 (OH) | 0.7 | 1.0 | 1.6E-01 | 4.5E-01 | 1.0 | 0.1 |
| Sul 42:3 | 2.9 | 3.6 | 3.8E-02 | 3.3E-04 | -1.9 | -3.6 |
| Sul 42:2 | 2.2 | 2.1 | 5.7E-02 | 5.1E-03 | -1.7 | -2.6 |
| Sul 42:1 | 1.2 | 1.4 | 2.8E-01 | 8.3E-02 | -0.6 | -1.4 |
| Sul 41:1 (OH) | 0.9 | 0.9 | 3.6E-01 | 3.6E-01 | 0.4 | 0.4 |
| Sul 41:0 (OH) | 0.8 | 0.9 | 2.3E-01 | 3.3E-01 | 0.8 | 0.5 |
| Sul 40:0 (2OH) | 0.7 | 0.9 | 1.7E-01 | 3.3E-01 | 1.0 | 0.5 |
| Sul 42:3 (OH) | 1.6 | 2.0 | 8.5E-02 | 6.7E-04 | -1.4 | -3.3 |
| Sul 41:0 (2OH) | 0.8 | 0.9 | 2.0E-01 | 3.9E-01 | 0.9 | 0.3 |
| Sul 43:2 (OH) | 1.5 | 1.9 | 1.2E-01 | 1.0E-03 | -1.2 | -3.2 |
| Sul 42:1 (2OH) | 0.8 | 1.1 | 2.0E-01 | 2.8E-01 | 0.9 | -0.6 |
| Sul 42:0 (2OH) | 0.8 | 1.0 | 2.8E-01 | 4.5E-01 | 0.6 | -0.1 |
| Sul 43:1 (2OH) | 1.7 | 2.1 | 7.7E-02 | 3.7E-04 | -1.5 | -3.5 |
| Sul 43:0 (2OH) | 1.4 | 1.3 | 1.8E-01 | 8.7E-02 | -0.9 | -1.4 |
| SulfoHex₂Cer 34:1 | 2.9 | 2.8 | 1.9E-02 | 2.6E-03 | -2.2 | -2.9 |
| SulfoHex₂Cer 34:1 (OH) | 2.1 | 1.8 | 5.4E-02 | 6.3E-03 | -1.7 | -2.6 |
| SulfoHex₂Cer 36:1 | 2.2 | 3.4 | 4.1E-02 | 1.8E-04 | -1.8 | -3.7 |
| SulfoHex₂Cer 38:1 | 3.1 | 3.4 | 4.2E-02 | 2.1E-03 | -1.8 | -3.0 |
| SulfoHex₂Cer 38:1 (OH) | 1.9 | 1.9 | 4.9E-02 | 5.5E-03 | -1.7 | -2.6 |
| SulfoHex₂Cer 40:2 | 3.0 | 3.8 | 3.7E-02 | 1.2E-03 | -1.9 | -3.2 |
| SulfoHex₂Cer 40:1 | 3.7 | 2.5 | 3.5E-02 | 2.4E-03 | -1.9 | -2.9 |
| SulfoHex₂Cer 41:1 | 2.3 | 2.7 | 4.3E-02 | 6.3E-05 | -1.8 | -4.0 |
| SulfoHex₂Cer 40:1 (OH) | 1.7 | 0.8 | 1.8E-01 | 2.0E-01 | -1.0 | 0.9 |
| SulfoHex₂Cer 42:3 | 2.6 | 3.5 | 3.9E-02 | 1.3E-03 | -1.9 | -3.1 |
| SulfoHex₂Cer 42:2 | 4.5 | 3.3 | 2.5E-02 | 1.1E-03 | -2.1 | -3.2 |
| SulfoHex₂Cer 42:1 | 3.3 | 2.6 | 3.5E-02 | 1.1E-03 | -1.9 | -3.2 |
| SulfoHex₂Cer 41:1 (OH) | 1.8 | 1.7 | 7.9E-02 | 1.0E-02 | -1.5 | -2.4 |
| SulfoHex₂Cer 40:0 (2OH) | 1.2 | 0.8 | 3.1E-01 | 2.2E-01 | -0.5 | 0.8 |

### Industrial Applicability

The method of the invention can be used for population screening of the whole population or selected population groups based on risk factors such as age, gender, body-mass-index, genetic predispositions, risk behavior, etc. The subjects having the positive output from population screening above a predetermined threshold can then be subjected to further examinations and tests (e.g., computer tomography or other advanced imaging methods). As the present screening method is non-invasive, can be performed in a high-throughput mode, and can detect early stages of cancers.

## Claims

1. A method of diagnosing kidney cancer based on lipidomic analysis of a body fluid sample selected from plasma, serum, blood or urine, taken from the body of a patient, **characterized in that** it comprises the steps of:
- spiking of the sample with a set of internal standards comprising at least one internal standard for each lipid class present in the sample,
- subsequently processing the sample by liquid-liquid lipidomic extraction or by solid phase lipidomic extraction,
- measurement of the processed sample by a mass spectrometry method,
- determining concentrations for at least 28, more preferably for all lipids present at a level above detection threshold of the mass spectrometry method, using the internal standards for the corresponding lipid classes for the quantitation, or using relative concentrations of lipids, or using ratios of concentrations and/or signal intensities,
- statistical evaluation of the determined concentrations of the lipids, said statistical evaluation determining the level of probability of the patient suffering from kidney cancer, based on the determined lipid concentrations compared to a cancerous pattern, and a non-cancerous pattern, wherein the cancerous pattern and the non-cancerous pattern are determined by statistical analysis of the lipid concentrations for a group of known cancer samples and non-cancer samples; and wherein the statistical evaluation is done separately for males and females.

2. The method according to claim 1, wherein the patient is a mammal, preferably a human.

3. The method according to any one of the preceding claims, wherein the internal standards are exogenous lipid compounds, having the polar head structure typical for the relevant lipid class and containing fatty acyls with shorter chains than naturally occurring lipids, preferably chains 12:0 or 14:0, or fatty acyls with odd number of carbon atoms, preferably chains 17:0, 17:1 or 19:1, or the internal standards are isotopically labelled analogues of the lipids of the relevant lipid class, preferably D7-Chol, D7-CE 16:0.

4. The method according to any one of the preceding claims, wherein a set of internal standards contains:
| | |
|---|---|
| D7-CE 16:0 | MG 19:1 |
| Cer d18:1/12:0 | PA 14:0/14:0 |
| DG 12:1/12:1 | PC 14:0/14:0 |
| Hex2Cer d18:1/12:0 | PE 14:0/14:0 |
| HexCer d18:1/12:0 | PG 14:0/14:0 |
| D7-Chol | PS 14:0/14:0 |
| LPC 17:0 | SM d18:1/12:0 |
| LPE 14:0 | SulfoHexCer d18:1/12:0 |
| LPG 14:0 | TG 19:1/19:1/19:1 |
| LPA 14:0 | LPS 17:1 |

5. The method according to any one of the preceding claims, wherein the mass spectrometry method is selected from shotgun mass spectrometry, ultrahigh-performance liquid chromatography - mass spectrometry, ultrahigh-performance supercritical fluid chromatography - mass spectrometry, and matrix-assisted laser desorption/ionization mass spectrometry.

6. The method according to any one of the preceding claims, wherein a pooled sample prepared by mixing identical volumes of several samples, wherein the mixed samples include samples from cancer patients and healthy volunteers, is used and processed in the same way as the measured samples, preferably the pooled sample is used for observing intra-day accuracy and intra-day precision, as well as inter-day accuracy and inter-day precision, and/or for determining the lower limit of quantitation and the upper limit of quantitation, and/or for quality control during measurements of sample set for all methods.

7. The method according to any one of the preceding claims, wherein the order of samples is randomized in sample measurement sequences.

8. The method according to any one of the preceding claims, wherein the step of determining concentrations for all lipids present at a level above lower limit of quantitation of the mass spectrometry method, using the internal standards for the corresponding lipid classes for the quantitation, comprises one or more of the following procedures and corrections:
- isotopic correction,
- zero filling procedure in which the signals of lipid species which are not detected for particular sample are replaced by 50 to 100 %, preferably by 60 to 100 % of the minimum concentration observed for said lipid species in all samples.

9. The method according to any one of the preceding claims, wherein the statistical evaluation involves
- data pre-processing such as centering, scaling, and/or transformation;
- PCA analysis for identification of influential factors such as outliers or measurement failures,
- discrimination analysis by OPLS-DA for group separation of cancer patients and healthy volunteers,
- assignment of the statistical parameters as well as the evaluation of the prediction power.

## Patentansprüche

1. Verfahren zur Diagnose von Nierenkrebs basierend auf der Lipidomanalyse einer Körperflüssigkeitsprobe, ausgewählt aus Plasma, Serum, Blut oder Urin, die von dem Körper eines Patienten entnommen wird, **dadurch gekennzeichnet, dass** es die folgenden Schritte umfasst:
- Dotieren der Probe mit einem Satz interner Standards, der mindestens einen internen Standard für jede in der Probe vorhandene Lipidklasse umfasst,
- anschließende Verarbeitung der Probe durch Flüssig-Flüssig-Lipidom-Extraktion oder durch Festphasen-Lipidom-Extraktion,
- Messung der verarbeiteten Probe durch ein Massenspektrometrieverfahren,
- Bestimmen der Konzentrationen für mindestens 28, bevorzugter für alle Lipide, die auf einem Niveau oberhalb der Nachweisgrenze des Massenspektrometrieverfahrens vorhanden sind, unter Verwendung der internen Standards für die entsprechenden Lipidklassen für die Quantifizierung oder unter Verwendung relativer Konzentrationen von Lipiden oder unter Verwendung von Verhältnissen von Konzentrationen und/oder Signalintensitäten,
- statistische Auswertung der ermittelten Konzentrationen der Lipide, wobei die statistische Auswertung den Grad der Wahrscheinlichkeit, dass der Patient an Nierenkrebs leidet, bestimmt, anhand der ermittelten Lipidkonzentrationen im Vergleich zu einem krebsartigen Muster und einem nicht krebsartigen Muster, wobei das krebsartige Muster und das nicht krebsartige Muster werden durch statistische Analyse der Lipidkonzentrationen für eine Gruppe bekannter krebsartiger proben und nicht krebsartiger Proben bestimmt; und wobei die statistische Auswertung getrennt für Männer und Frauen erfolgt.

2. Verfahren nach Anspruch 1, wobei der Patient ein Säugetier, vorzugsweise ein Mensch, ist.

3. Verfahren nach einem der vorhergehenden Ansprüche, wobei es sich bei den internen Standards um exogene Lipidverbindungen handelt, die die für die jeweilige Lipidklasse typische Polkopfstruktur aufweisen und Fettacyle mit kürzeren Ketten als natürlich vorkommende Lipide enthalten, vorzugsweise Ketten 12:0 oder 14:0, oder Fettacyle mit ungerader Anzahl an Kohlenstoffatomen, vorzugsweise Ketten 17:0, 17:1 oder 19:1, oder die internen Standards sind isotopenmarkierte Analoga der Lipide der relevanten Lipidklasse, vorzugsweise D7-Chol , D7-CE 16:0.

4. Verfahren nach einem der vorhergehenden Ansprüche, wobei der Satz interner Standards enthält:
| | |
|---|---|
| D7-CE 16:0 | MG 19:1 |
| Cer d18:1/12:0 | PA 14:0/14:0 |
| DG 12:1/12:1 | PC 14:0/14:0 |
| Hex2Cer d18:1/12:0 | PE 14:0/14:0 |
| HexCer d18:1/12:0 | PG 14:0/14:0 |
| D7-Chol | PS 14:0/14:0 |
| LPC 17:0 | SM d18:1/12:0 |
| LPE 14:0 | SulfoHexCer d18:1/12:0 |
| LPG 14:0 | TG 19:1/19:1/19:1 |
| LPA 14:0 | LPS 17:1 |

5. Verfahren nach einem der vorhergehenden Ansprüche, wobei das Massenspektrometrieverfahren ausgewählt ist aus Shotgun-Massenspektrometrie, Ultrahochleistungs-Flüssigkeitschromatographie - Massenspektrometrie, Ultrahochleistungs-superkritischer-Flüssigkeitschromatographie - Massenspektrometrie und Matrix-unterstützter Laserdesorption/Ionisationsmassenspektrometrie.

6. Verfahren nach einem der vorhergehenden Ansprüche, wobei eine gepoolte Probe, hergestellt durch Mischen identischer Volumina mehrerer Proben, wobei die gemischten Proben Proben von Krebspatienten und gesunden Freiwilligen umfassen, in gleicher Weise wie die gemessenen Proben verwendet und verarbeitet wird, vorzugsweise wird die gepoolte Probe zur Beobachtung der Intra-Day-Genauigkeit und Intra-Day-Präzision sowie der Inter-Day-Genauigkeit und Inter-Day-Präzision, und/oder zur Bestimmung der unteren Quantifizierungsgrenze und der oberen Quantifizierungsgrenze, und/oder zur Qualitätskontrolle bei Messungen des Probensatzes für alle Methoden, verwendet.

7. Verfahren nach einem der vorhergehenden Ansprüche, wobei die Reihenfolge der Proben in Probenmesssequenzen randomisiert wird.

8. Verfahren nach einem der vorhergehenden Ansprüche, wobei der Schritt der Bestimmung der Konzentrationen für alle Lipide, die auf einem Niveau oberhalb der unteren Quantifizierungsgrenze des Massenspektrometrieverfahrens vorhanden sind, unter Verwendung der internen Standards für die entsprechenden Lipidklassen für die Quantifizierung, umfasst einen oder mehrere der folgenden Eingriffe und Korrekturen:
- Isotopenkorrektur,
- Nullfüllverfahren, bei dem die Signale von Lipidspezies, die für eine bestimmte Probe nicht nachgewiesen werden, durch 50 bis 100 %, vorzugsweise durch 60 bis 100 %, der für diese Lipidspezies in allen Proben beobachteten Mindestkonzentration ersetzt werden.

9. Verfahren nach einem der vorhergehenden Ansprüche, wobei die statistische Auswertung erfolgt
- Datenvorverarbeitung wie Zentrierung, Skalierung und/oder Transformation;
- PCA-Analyse zur Identifizierung von Einflussfaktoren wie Ausreißern oder Messfehlern,
- Diskriminierungsanalyse durch OPLS-DA zur Gruppentrennung von Krebspatienten und gesunden Freiwilligen,
- Zuweisung der statistischen Parameter sowie Bewertung der Vorhersagekraft.

## Revendications

1. Procédé de diagnostic du cancer du rein basé sur l'analyse lipidomique d'un échantillon de fluide corporel choisi parmi le plasma, le sérum, le sang ou l'urine, prélevé sur le corps d'un patient, **caractérisé en ce qu'**il comprend les étapes de :
- dopage de l'échantillon avec un jeu de standards internes comprenant au moins un standard interne pour chaque classe lipidique présente dans l'échantillon,
- traitement ultérieur de l'échantillon par extraction lipidomique liquide-liquide ou par extraction lipidomique en phase solide,
- mesure de l'échantillon traité par une méthode de spectrométrie de masse,
- déterminer des concentrations pour au moins 28, plus préférablement pour tous les lipides présents à un niveau supérieur au seuil de détection de la méthode de spectrométrie de masse, en utilisant les étalons internes pour les classes de lipides correspondantes pour la quantification, ou en utilisant des concentrations relatives de lipides, ou en utilisant des rapports de concentrations et/ou intensités des signaux,
- évaluation statistique des concentrations déterminées des lipides, ladite évaluation statistique déterminant le niveau de probabilité que le patient souffre d'un cancer du rein, sur la base des concentrations déterminées de lipides par rapport à un schéma cancéreux, et un schéma non cancéreux, où le schéma cancéreux et le schéma non cancéreux sont déterminés par analyse statistique des concentrations de lipides pour un groupe d'échantillons cancéreux connus et d'échantillons non cancéreux connus; et où l'évaluation statistique est effectuée séparément pour les hommes et les femmes.

2. Procédé selon la revendication 1, où le patient est un mammifère, de préférence un humain.

3. Procédé selon l'une quelconque des revendications précédentes, où les standards internes sont des composés lipidiques exogènes, ayant la structure de tête polaire typique de la classe lipidique concernée et contenant des acyles gras avec des chaînes plus courtes que les lipides naturels, de préférence des chaînes 12:0. ou 14:0, ou des acyles gras avec un nombre impair d'atomes de carbone, de préférence des chaînes 17:0, 17:1 ou 19:1, ou les standards internes sont des analogues isotopiquement marqués des lipides de la classe de lipides concernée, de préférence D7-Chol, D7-CE 16:0.

4. Procédé selon l'une quelconque des revendications précédentes, où le jeu de standards internes contient :
| | |
|---|---|
| D7-CE 16:0 | MG 19:1 |
| Cer d18:1/12:0 | PA 14:0/14:0 |
| DG 12:1/12:1 | PC 14:0/14:0 |
| Hex2Cer d18:1/12:0 | PE 14:0/14:0 |
| HexCer d18:1/12:0 | PG 14:0/14:0 |
| D7-Chol | PS 14:0/14:0 |
| LPC 17:0 | SM d18:1/12:0 |
| LPE 14:0 | SulfoHexCer d18:1/12:0 |
| LPG 14:0 | TG 19:1/19:1/19:1 |
| LPA 14:0 | LPS 17:1 |

5. Procédé selon l'une quelconque des revendications précédentes, où le procédé de spectrométrie de masse est choisi parmi la spectrométrie de masse shotgun, la chromatographie liquide ultra haute performance - spectrométrie de masse, la chromatographie fluide supercritique ultra haute performance - spectrométrie de masse, et la désorption laser assistée par matrice/spectrométrie de masse à ionisation.

6. Procédé selon l'une quelconque des revendications précédentes, où un échantillon groupé préparé en mélangeant des volumes identiques de plusieurs échantillons, où les échantillons mélangés comprennent des échantillons de patients cancéreux et de volontaires sains, est utilisé et traité de la même manière que l'échantillon mesuré; de préférence l'échantillon groupé est utilisé pour observer l'exactitude intra-journalière et la précision intra-journalière, ainsi que l'exactitude inter-journalière et la précision inter-journalière, et/ou pour déterminer la limite inférieure de quantification et la limite supérieure de quantification, et/ou pour le contrôle de la qualité lors des mesures de l'ensemble d'échantillons pour toutes les méthodes.

7. Procédé selon l'une quelconque des revendications précédentes, où l'ordre des échantillons est randomisé dans des séquences de mesure d'échantillons.

8. Procédé selon l'une quelconque des revendications précédentes, où l'étape de détermination des concentrations pour tous les lipides présents à un niveau supérieur à la limite inférieure de quantification du procédé de spectrométrie de masse, en utilisant les étalons internes pour les classes de lipides correspondantes pour la quantification, comprend une ou plusieurs des procédures et corrections suivantes :
- correction isotopique,
- procédure de remplissage zéro dans laquelle les signaux d'espèces lipidiques qui ne sont pas détectés pour un échantillon particulier sont remplacés par 50 à 100 %, de préférence par 60 à 100 %, de la concentration minimale observée pour ladite espèce lipidique dans tous les échantillons.

9. Procédé selon l'une quelconque des revendications précédentes, où l'évaluation statistique contient:
- le prétraitement des données tel que le centrage, la mise à l'échelle et/ou la transformation ;
- analyse PCA pour l'identification des facteurs influents tels que les valeurs aberrantes ou les échecs de mesure,
- analyse de discrimination par l'OPLS-DA pour la séparation des groupes de patients cancéreux et de volontaires sains,
- l'affectation des paramètres statistiques ainsi que l'évaluation du pouvoir de prédiction.
